# EUROPEAN PATENT SPECIFICATION

(11) **EP 2 826 856 B1**
(45) Date of publication and mention of the grant of the patent: **23.12.2015**
(21) Application number: 13176757.6
(22) Date of filing: 16.07.2013
(51) Int. Cl.: C12N 7/00, A61K 35/76

(54) **Genetically stable oncolytic RNA virus, method of manufacturing and use thereof**
Genetisch stabiles onkolytisches RNA-Virus, Verfahren zur Herstellung und Verwendung davon
Virus d'ARN oncolytique génétiquement stable, son procédé de fabrication et utilisation associée

(43) Date of publication of application: 21.01.2015
(73) Proprietor: Sia Latima, 2114 Olaine (LV)
(72) Inventor: Venskus, Dite, LV-3008 Jelgava (LV); Kalvins, Ivars, LV-1006 Riga (LV); Pjanova, Dace, LV-1057 Riga (LV); Petrovska, Ramona, LV-1083 Riga (LV); Auzins, Jurgis, LV-2114 Olaine (LV)
(74) Representative: Madgwick, Paul Roland

(56) References cited:
- EP-A1- 1 537 872
- FERDAT A K: "MECHANISM OF IMMUNOMODULATION IN THE ANTI-TUMOUR EFFECT OF THE ECHO-7 ENTEROVIRUS", EKSPERIMENTAL NAA ONKOLOGIA - EXPERIMENTAL ONCOLOGY, KIEV, UU, vol. 11, no. 5, 1 January 1989 (1989-01-01), pages 43-48, XP008054566, ISSN: 0204-3564
- CHUA B H ET AL: "Comparison of the complete nucleotide sequences of echovirus 7 strain UMMC and the prototype (Wallace) strain demonstrates significant genetic drift over time", November 2001 (2001-11), JOURNAL OF GENERAL VIROLOGY, VOL. 82, NR. 11, PAGE(S) 2629-2639, XP002717948, ISSN: 0022-1317 * page 2629 *

## Description

### TECHNICAL FIELD

The invention relates to development of a novel biotechnologically produced anti-cancer preparation, namely to a genetically stable oncolytic RNA virus, a method for manufacturing the oncolytic virus, and use thereof.

### BACKGROUND ART

The ability of viruses to kill cancer cells is known for more than a century [Kelly, E.; Russell, S.J. History of oncolytic viruses: genesis to genetic engineering. Mol. Ther. 2007, 15, pp. 651-659] and there were numerous promising successes in experimental cancer therapy with various viruses, nevertheless their use in clinical practice is hampered by the difficulty to foresee the interaction between the tumour and its host, as well as the virus and response of human immune system to viral antibodies.

Although the clinical investigations regarding the use of viruses in cancer therapy commenced more than 50 years ago, at present only two viruses are approved for clinical use in cancer therapy. They are adenovirus with deleted E1 B 55K gene (Garber, K. China approves world's first oncolytic virus therapy for cancer treatment. J. Natl. Cancer Inst. 2006, 98, pp. 298-300) and unmodified passivized Picornaviridae Enterovirus of Echo type (Eurasian patent 007839; European patent application 03733607), acting as antitumour immunostimulant.

EP 1537872 discloses the use of an ECHO-7 virus in the active immunotherapy of tumours. Virus was recovered from the intestinal tract, and isolated in a human primary embryonic cell culture. Page 2, paragraph 18 states that nonpathogenic serotypes of virus (in particular ECHO 7), were selected. Virus was cultured in tumour tissue, then in human embryonic fibroblasts, and the process repeated. The virus obtained was demonstrated as being able to improve survival in patients with skin melanomas.

The development of novel efficient oncolytic viruses is still a topical problem (Han Hsi Wong, Nicholas R. Lemoine,Yaohe Wang, Viruses 2010, 2, pp. 78-106).

In order to increase the potential of virus so selectively infect cancer cells and heighten the oncolytic activity, a number of modified viruses have been disclosed. They are characterised by deletion of specific genes, thus preventing their propagation in normal cells, or integration of additional genes for improving the oncolytic properties.

However, the limited knowledge concerning the genetical modifications that provide for selectivity and efficiency against the tumour cells, results in modified viruses with lower cytolytic activity, compared to origin, or higher anti-virus response of human immune system (S.Meerani, Yang Yao, Oncolytic viruses in cancer therapy. European Journal of Scientific Research, vol. 40 no.1 (2010), pp.156-171; Han Hsi Wong, Nicholas R. Lemoine,Yaohe Wang, Viruses 2010, 2, 78-106).

Although viruses are well-established tools for conveying vectors into cell, their use is limited by the high immunogenicity of viruses (Peng, Z. Current status of gendicine in China: recombinant human Ad-p53 agent for treatment of cancers. Hum. Gene. Ther. 2005, 16, 1016-1027).

One of the most serious adverse properties of non-modified ECHO type viruses, including ECHO 7, is their ability to cause infections that may have a fatal result (Wreghitt T.G., Gandy G.M., King A., Sutehall G., Fatal neonatal ECHO 7 virus infection, The Lancet, vol. 324, p.465, 1984). These viruses are known to be responsible for hand, foot and mouth disease in Malaysia (http://www.vadscorner.com/echovirus7.html), for myocarditis in leukemic child (Midula M., Marzetti G., Borra G., Sabatino G., Myocarditis associated with ECHO 7 type infection in leukemic child, Acta Paediatrica Volume 65, Issue 4, pp. 649-651, July 1976), aseptic meningitis, paralytic disease and fever (http://virology-online.com/viruses/Enteroviruses6.htm).

Therefore pathogenicity is one of the major limitations that must be overcome in using ECHO 7 type viruses in treating cancer patients.

### DISCLOSURE OF THE INVENTION

### [TECHNICAL PROBLEM]

Therefore, the problem to solve was the development a highly efficient, selective oncolytic virus without pathogenicity in normal cells and low immunological response, and possessing high genetic stability.

### [SOLUTION TO PROBLEM]

This problem was surprisingly solved by a targeted modification of a single-strand RNA virus by developing a method that utilized the high mutation potential of single strand RNA virus in combination with a specifically targeted selection of mutants, providing for fast separation from the pool of mutant species with high and selective oncolytic activity. Many cancer cells are resistant to the virus (the virus can not enter the cell and survive there). By careful selection of cell lines where the virus is modified and by proper pretreatment of the cancer cells it is possible to create a genetically stable and non-pathogenic virus for cancer treatment.

### SHORT DESCRIPTION OF THE INVENTION

We have developed a method for modifying the native ECHO 7 virus, identified by genome sequence SeqNo2, the method comprising initially conducting the virus adaptation in cancer cells, attenuated by an anti-cancer agent such as dacarbazine, passaging the modified virus in human embryonal fibroblast culture, propagation in human melanoma cells and passaging in human embryonal fibroblast culture, optionally treated by ribavirin, isolation of the virus and purification of the virus. The virus can be isolated and purified by known methods.

More than one type of cell lines can be used during conducting the virus adaptation.

The invention is as set out in claims 1-12.

### BRIEF DESCRIPTION OF THE APPENDICES CONTAINING THE SEQUENCES (which appendices are part of the description)

Appendix 1: Nucleotide sequence of the modified virus(Seq ID No 1)
Appendix 2: Nucleotide sequence of the unmodified (native) virus (Seq ID No 2)
Appendix 3. Nucleotide sequence of the modified virus after propagation for 12 months (Seq ID No 3)
Appendix 4. Comparison of genomes of the modified virus and unmodified (native) virus (Seq ID No 4)
Appendix 5. Comparison of amino acid sequences of the modified virus and unmodified (native) virus (Seq ID No 5).

### DETAILED DESCRIPTION OF THE INVENTION

We have unexpectedly discovered the suitability for this purpose of a known Echo 7 type *Picornaviridae* enterovirus, isolated from a human intestine. The original nucleotide sequence, determined by a standard method, was found to be rather similar to that of Wallace type *Picornaviridae Enterovirus.*

Checking the oncolytic activity of isolated native enteroviruses in tissue of angiosarcoma demonstrated that neither individual viruses nor their combinations in a dose 3x10⁵ TCID₅₀/0.03 ml possessed substantial oncolytic activity with exception of ECHO 7 type that showed more promising activity (Table 1).

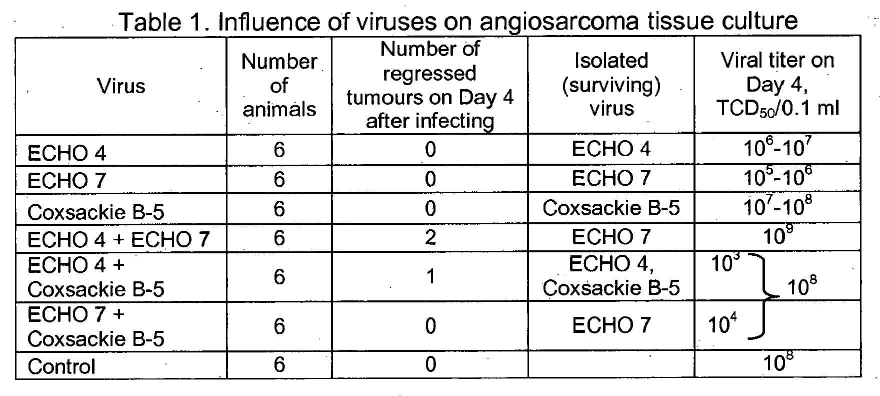

The instability of the genome of the RNA single strand viruses is a well-known fact; therefore, such viruses usually are not selected for constructing oncolytic viral agents.

The modification of the isolated native virus was realised in several consecutive steps.

The first step takes advantage of the high mutation potential of RNA viruses (on average one mutation on each replication) to develop a cytopathic mutant by replicating the virus in trypsinized monolayer of human embryonic fibroblast culture in presence of calf serum.

Cells were incubated for 10 days, carrying out the passage each time when the cells in culture had degenerated for 50%.

Testing the selected virus in RD cell culture a pronounced cytopathic effect was observed already in 24 hours after infection. The titer of the developed virus, determined by last dilution method was TCID₅₀=1 x 10⁻⁸.

This strain was propagated, isolated and stored at -70 °C for further use in producing selective and genetically stable oncolytic strain.

The virus so obtained was modified, using specially developed method comprising three steps.

### 1st modification step in a first tumour cell line

In the first modification step, the virus was propagated in tumour cell lines attenuated by anticancer agent. Human breast adenocarcinoma cell line (MCF-7) was used in this step.

A monolayer of these cells was treated with dacarbazine DTIC in sub toxic dose (20 µM). After treating with dacarbazine, the cells were transferred to fresh culture medium and contacted with the virus, and the propagation continued without adding serum. After 24 hours from contacting with the virus, the cells were removed and the virus was isolated from the media.

The virus was repeatedly propagated (passaged) in human embryonic fibroblast cell culture and again used for infecting the MCF-7 cell line. This procedure was repeated 10 times. Thus, this modification step comprises alternately propagating the virus in human breast adenocarcinoma cells and human embryonic fibroblast cells.

### 2nd modification step in a second tumour cell line

In the next modification step, the virus as described above, was contacted with gastric adenocarcinoma cell culture. A monolayer of these cells was treated with dacarbazine DTIC in sub toxic dose (20 µM).

The monolayer of these cells was infected by the virus, which was isolated after the modification in the first step, and the propagation continued in a culture medium without serum.

After 24 hours from contacting with the virus, the cells were removed and virus isolated from the media. The virus was repeatedly propagated (passaged) in human embryonic fibroblast cell culture, and thereafter again used for infecting the gastric adenocarcinoma cell line. This procedure was repeated 10 times. Thus, this second modification step comprises alternately propagating the virus in gastric adenocarcinoma cells and in human embryonic fibroblast cells.

In the first modification step and in the second modification step, the propagation procedure was always finished by propagating the virus last in the human embryonic fibroblast culture.

### 3rd modification step in human melanoma cells

The virus produced in the second step was used for infecting human tumours, obtained in surgery.

Melanoma cancer tissues were obtained in surgery from 23 patients previously treated by chemotherapy.

Tissues were infected by the modified virus and incubated at 37 °C in the absence of carbon dioxide. Before being used for infecting a new tissue material (fresh melanoma tissue from another patient), the modified virus was repeatedly propagated in human embryonic fibroblast culture to titer 7 lg TCID ₅₀/1 ml.

The modified virus was propagated in human embryonic fibroblast cell culture that was treated by 5 mM ribavirin 7 hours before infection and cultivated for 24 hours. The virus was isolated from the culture, and the procedure of propagating the virus in the fibroblast culture was repeated 10 times.

Finally, the virus was isolated, purified and propagated in human embryonic fibroblast culture without addition of ribavirin.

The propagated virus was used for sequence determination. The genome of the modified virus differs from that of frozen unmodified native virus (Echo 7 type Wallace strain from NCBI database) for about 10%, the coat part for about 12%.

The virus modified by the described method was found to be surprisingly stable. Its genome changed for only 0.7% after continuous passaging for 12 months (propagation for 12 months in human embryonal lung culture MRC 5).

Especially important is the fact that the modified virus (further on MV) is characterised by exceptionally high cytopathic effect on malignant cells and low cytotoxicity on normal cell lines as well as no toxicity *in vivo* in mice.

In experiments with cell lines MV was found to be cytotoxic for melanoma cell lines FM9, FM55, FM94 and SK-Mel26, gastric carcinoma cells, human oral squamous cell carcinoma SCC25 cells, human epithelial cell line derived from a lung carcinoma (A549), acute monocyte leukemia THP-1 cells, rabdomyosarcoma RD cells, human pancreatic adenocarcinoma HPAF-II cells, human breast adenocarcinoma cells (MCF-7) as well as on primary cell cultures of gastric adenocarcinoma GC1 and thyroid cancer line HA007.

In animal experiments, MV caused regression of murine sarcoma M-1, mice fibrosarcoma MX-17 as well as transplantable tumours - Moloney sarcoma (SM) and KRS-321 sarcoma.

In a clinical pilot study, a group of 46 melanoma stage I patients no progress of melanoma was observed for 50 months in 43 patients, treated with MV. In the control group, melanoma progressed for 10 of 31 patients undergoing standard therapy.

In a 50 months study of 44 stage II melanoma patients the progress of melanoma was stopped in 38 patients, compared to control group of 36 patients undergoing standard therapy, where melanoma did not progress in 15 patients, but did progress in 21 patient. No serious adverse effects were observed for patients treated with MV.

### INDUSTRIAL APPLICABILITY

We have developed a novel virus strain (MV) with original genome sequence, stable against genetic drift, possessing cytopathic activity against various types of tumours, characterized by low incidence of adverse effects and low toxicity that can be used with advantage in cancer virotherapy. Thus, we have unexpectedly solved the main obstacle in wider use of RNA viruses in medicine - obtained genetically stable strain that can be used in standardized continuous manufacturing of oncolytic viral preparation. The viral preparation can be used in anticancer therapy against a variety of tumour cells.

### EXAMPLES

The present invention is described in Examples in more detail. However, the invention is not construed as being limited to the examples.

### Virus

The virus modified according to the invention is ECHO-7 virus (Picornaviridae family, Enterovirus genus, ECHO (Enteric Cytopathic Human Orphan) type 7, group IV, positive-sense single stranded RNA virus).

### Example 1. The isolation and characterization of the original virus strain

A known method for isolation (A.C. Rentz, J.E. Libbey, R.S. Fujinami, F.G. Whitby, and C.L. Byington. Investigation of Treatment Failure in Neonatal Echovirus 7 Infection. The Pediatric Infectious Disease Journal, Volume 25, Number 3, March 2006, 259) and propagation in BS-C-1 cell line (CCL 26; ATCC) was used (Libbey JE, McCright IJ, Tsunoda I, et al. Peripheral nerve protein, P0, as a potential receptor for Theiler's murine encephalomyelitis virus. J Neurovirol. 2001;7:97-104. Pevear DC, Tull TM, Seipel ME, et al. Activity of pleconaril against enteroviruses. Antimicrob Agents Chemother. 1999;43:2109-2115). Virus propagation and determination of titer was conducted in concordance with the published method (Zurbriggen A, Fujinami RS. A neutralization-resistant Theiler's virus variant produces an altered disease pattern in the mouse central nervous system. J Virol. 1989;63:1505-1513).

### Example 2. Virus modification

In the first modification step, the virus was propagated in tumour cell lines attenuated by an anticancer agent. Initially, for propagation was used the human breast adenocarcinoma cell culture (MCF-7), cultivated in DME medium (Sigma-Aldrich) with 10% serum (Gibco) and antibiotics (100 IU/ml penicillin, 100 IU/ml streptomycin) at 37 °C under atmosphere, containing 5% CO₂ untill developing of the monolayer.

The obtained monolayer of these cells was treated with dacarbazine DTIC in sub toxic dose (20 µM). After treating with dacarbazine cells were transferred to fresh culture medium without added serum, the cells contacted with virus and the propagation continued.

After 24 hours from contacting with the virus the cells were removed and virus isolated from the media. The virus was repeatedly propagated in human embryonal fibroblast cell culture and again used for infecting the MCF-7 cell line. This procedure was repeated 10 times.

In the next, second step, the virus as described above, was contacted with gastric adenocarcinoma cell culture. The cell culture for propagation was cultivated in DME medium (Sigma-Aldrich) with 10% serum (Gibco) and antibiotics (100 IU/ml penicillin, 100 IU/ml streptomycin) at 37 °C under atmosphere, containing 5% CO₂ untill developing of the monolayer.

The obtained monolayer of these cells was treated with dacarbazine DTIC in sub toxic dose (20 µM). After treating with dacarbazine cells were transferred to fresh culture medium without added serum, the cells contacted with virus and the propagation continued.

After 24 hours from contacting with the virus the cells were removed and virus isolated from the media. The virus was repeatedly propagated in human embryonal fibroblast cell culture and again used for infecting the gastric adenocarcinoma cell line. This procedure was repeated 10 times.

In the third step, the virus produced in the second step was used for infecting human tumours, obtained in surgery. Melanoma cancer tissues were obtained in surgery from 23 patients previously treated by chemotherapy.

The tumour cells were separated from fat cells, necrotic tissue and blood, kept at 0 °C for 24 hours, fragmented and as approximately 0.1 cm³ large tissue pieces immersed in Eagle medium (4 ml of medium for 10 mg of tissue), infected with the prepared virus and incubated in the absence of carbon dioxide at 37 °C.

The medium was replaced by a fresh portion every day until the destruction of tumour, determined morphologically and visually by the oxidation level of medium.

The virus titer was determined every day in tumor tissue fee medium sample. The reproduction rate of virus was determined from the virus titer at the conclusion of an experiment in comparison with that on Day 0. Such modification of virus was performed in tissues obtained from 23 patients.

Before being used for infecting a new tissue material, the modified virus was each time repeatedly propagated in human embryonal fibroblast culture to titer 7 Ig TCID ₅₀/1⁻ml.

The modified virus was propagated in human embryonal fibroblast cell culture that was treated by 5 mM ribavirin 7 hours before infection and cultivated for 24 hours. Virus was isolated from culture medium, and the procedure repeated 10 times.

Finally, the virus was isolated, purified and propagated in human embryonal fibroblast culture without addition of ribavirin.

The propagated virus sample was used for determination of genome sequence, anticancer activity and replicative stability by passaging it for 12 months in human embryonal fibroblast culture with repeated determination of genome sequence (Appendix 1).

### Example 3. Determination of virus genome sequence

The isolation, amplification and sequencing of the isolated, modified and cultivated virus genome were performed according to the known method [Chua BH, McMinn PC, Lam SK, Chua KB. Comparison of the complete nucleotide sequences of echovirus 7 strains UMMC and the prototype (Wallace) strain demonstrates significant genetic drift over time. J Gen Virol. 2001 Nov; 82(Pt 11): 2629-39].

For this purpose, 96 enteroviruses with complete genome sequence were selected from the NCBI Gene bank. The complete genome sequences for these viruses were downloaded and compared by Vector NTI program.

Based on the results of comparing the most conservative regions of virus genomes were determined and 13 degenerated oligonucleotide pairs selected in these regions, covering the length of the potential enteroviruses genome. After the synthesis of the first 13 fragments, another 13 nucleotide pairs were produced. These oligonucleotide pairs were virus-specific and designed so as to produce overlaying fragments. After the building of the full genome sequence the virus genome was repeatedly sequenced with the virus-specific primers.

### Example 3.1. The genome sequence of the unmodified (native) virus

The sequence of the native virus was produced from 26 separate overlapping PCR fragments, synthesized from the primers listed in Table 2.

**Table 2. Primers used to sequence the complete genome of viruses.**

| **Primer** | **Sequence (5'- 3')** | **Length (bp)** | **Position** | **Target region** |
|---|---|---|---|---|
| **Eo7-1F** | **TTAAAACAGCCTGTGGGTTG** | **20** | **1-20** | **5'UTR** |
| **Eo7-1R** | **GAAACACGGACACCCAAAGTAG** | **22** | **545-566** | **5'UTR** |
| **Eo7-2F** | **CCATGGGACGCTTCAATACT** | **20** | **391-410** | **5'UTR** |
| **Eo7-2R** | **GCACCAGTCTTTTGTGTCGA** | **20** | **758-777** | **VP4** |
| **Eo7-3F** | **CGACTACTTTGGGTGTCCGTGTTTC** | **25** | **542-566** | **5' UTR** |
| **Eo7-3R** | **TCDGGRAAYTTCCACCACCACCC** | **23** | **1178-1200** | **VP2** |
| **Eo7-4F** | **CGACAGGGTGAGATCCCTAA** | **20** | **979-998** | **VP2** |
| **Eo7-4R** | **TTTCACCCTTCGTGAGGTTC** | **20** | **1381-1400** | **VP2** |
| **Eo7-5F** | **GCATCYAARTTYCAYCARGG** | **20** | **1289-1308** | **VP2** |
| **Eo7-5R** | **CACATKGGKGCAATSGTGAC** | **20** | **1676-1695** | **VP2** |
| **Eo7-6F** | **GTGGATCAACTTGCGCACTA** | **20** | **1513-1532** | **VP2** |
| **Eo7-6R** | **AAATTGTGGCATAGCCGAAG** | **20** | **1797-1816** | **VP3** |
| **Eo7-7F** | **GTCACSATTGCMCCMATGTG** | **20** | **1676-1695** | **VP2** |
| **Eo7-7R** | **CTTNATRCTYCCTGACCAGTGTG** | **23** | **2055-2077** | **VP3** |
| **Eo7-8F** | **AAGCATGGACGCATATCACA** | **20** | **1921-1940** | **VP3** |
| **Eo7-8R** | **GATATGGGTTCCCACATTGC** | **20** | **2174-2194** | **VP3** |
| **Eo7-9F** | **CACACTGGTCAGGRAGYATNAAG** | **23** | **2055-2077** | **VP3** |
| **Eo7-10F** | **CAAGTGTGTCGTCCTGTGCT** | **20** | **2350-2369** | **VP3** |
| **Eo7-9R** | **CCTATTGGCGCTGTCTTGAT** | **20** | **2694-2713** | **VP1** |
| **Eo7-11F** | **ACCAAAGATCAAGACAGCGC** | **20** | **2687-2706** | **VP1** |
| **Eo7-11R** | **TTGGCACCCACACTCTGATA** | **20** | **3178-3197** | **VP1** |
| **Eo7-12F** | **ACCAGTCCGGTGCTGTTTAC** | **20** | **3336-3355** | **VP1-2A** |
| **Eo7-12R** | **TCCCAYACACARTTYTGCCAGTC** | **23** | **3401-3423** | **2A** |
| **Eo7-13F** | **CARAAYTGTGTGTGGGAAGACTA** | **23** | **3407-3429** | **2A** |
| **Eo7-13R** | **CCCTGYTCCATKGCTTCATCYTCYARC** | **27** | **3748-3774** | **2A-2B** |
| **Eo7-14F** | **TTACCCAGTCACCTTCGAGG** | **20** | **3535-3554** | **2A** |
| **Eo7-14R** | **TGTTTTTCCTTCACTTCCGG** | **20** | **4181-4200** | **2C** |
| **Eo7-15F** | **GTTRGARGATGATGCNATGGARCARGG** | **27** | **3748-3774** | **2A-2B** |
| **Eo7-15R** | **TCAATACGGYRTTTGSWCTTGAA** | **23** | **4409-4431** | **2C** |
| **Eo7-16F** | **CCTYTRTAYGCVGCYGARGC** | **20** | **4343-4362** | **2C** |
| **Eo7-17F** | **TTCAAGWSCAAAYRCCGTATTGA** | **23** | **4409-4431** | **2C** |
| **Eo7-16R** | **AAYTGAATGGCCTTHCCACACAC** | **23** | **4922-4944** | **2C** |
| **Eo7-18F** | **CTDGTGTGTGGRAAGGCYATNCA** | **23** | **4919-4941** | **2C** |
| **Eo7-18R** | **TATGCTCCYTGRAARCCTGCAAA** | **23** | **5309-5330** | **3A-3B** |
| **Eo7-19F** | **CAAGCCCTAACCACGTTTGT** | **20** | **5252-5271** | **3A** |
| **Eo7-19R** | **ACCCGTAGTCAGTCACCTGG** | **20** | **5740-5759** | **3C** |
| **Eo7-20F** | **TTTGCAGGMTTYCARGGWGCATA** | **23** | **5309-5330** | **3A-3B** |
| **Eo7-20R** | **GCYCTWGTGGGRAAGTTRTACAT** | **23** | **5723-5745** | **3C** |
| **Eo7-21F** | **GTGTTGGATGCCAAGGAACT** | **20** | **5555-5574** | **3C** |
| **Eo7-21R** | **ATGGGCTCCGATCTGATGTC** | **20** | **6203-6222** | **3D** |
| **Eo7-22F** | **TTCCCCACWAGRGCAGGCCARTGYGG** | **26** | **5907-5832** | **3C** |
| **Eo7-22R** | **CTCCAAAABASRTCYGGGTCRCA** | **23** | **6572-6594** | **3D** |
| **Eo7-23F** | **TGAAGGAATGCATGGACAAA** | **20** | **6360-6379** | **3D** |
| **Eo7-23R** | **ATGGGTATTGCTCATCTGCC** | **20** | **7078-7097** | **3D** |
| **Eo7-24F** | **TGYGACCCRGAYSTVTTTTGGAG** | **23** | **6572-6594** | **3D** |
| **Eo7-24R** | **TCRTGDATDTCYTTCATGGGCA** | **22** | **7116-7137** | **3D** |
| **Eo7-25F** | **CCTGGACGAATGTGACCTTT** | **20** | **7041-7060** | **3D** |
| **Eo7-25R** | **CCCTACCGCACTTTTATCCA** | **20** | **7384-7403** | **3'UTR** |
| **Eo7-26F** | **ATCCAYGARTCHATYAGRTGGAC** | **23** | **7130-7152** | **3D** |
| **Eo7-26R** | **CCGCACCGAATGCGGAGAATTTAC** | **24** | **7404-7427** | **3'UTR** |

| | | | | |
|---|---|---|---|---|
| **UTR - untranslated region.** | | | | |

The 5'-terminal and the 3'-terminal sequences were obtained, using 5'-RACE and 3'-RACE methods, correspondingly.

As a result, the full genome sequence of the unmodified virus was found to consist of 7434 nucleotides, excluding the poly A sequence (Appendix 2; Seq ID No 2).

The untranslatable 5'-terminal (5'NTR) contains 742 nucleotides, followed by coding part starting with start codon (AUG) at position 743, containing codons for 2196 amino acids and ending with stop codon (UAA) at position 7331 (Appendix 2). The untranslatable 3'-terminal (3'NTR) of this strain contains 100 nucleotides, followed by poly A sequence.

### Example 3.2. The sequence of the modified virus (MV)

The sequence of the starting virus was produced from 26 separate overlapping PCR fragments, synthesized using the primers listed in Table 2.

The 5'-terminal and the 3'-terminal sequences were obtained, using 5'-RACE and 3'-RACE methods, correspondingly.

As a result, the full genome sequence of the modified virus was found to consist of 7427 nucleotides, excluding the poly A sequence (Appendix 1; Seq ID No 1).

The untranslatable 5'-terminal (5'NTR) of this strain contains 742 nucleotides, followed by the coding sequence. The coding part that contains information about the virus polyprotein, begins with the start codon (AUG) at position 743, contains codons for 2194 amino acids and ends with stop codon (UAA) at position 7325 (Appendix 1). The untranslatable 3'-terminal (3'NTR) of this strain contains 100 nucleotides, followed by poly A sequence.

### Example 3.3. The genome sequence of the modified virus after propagation for 12 months

The sequence of the modified virus was produced from 26 separate overlapping PCR fragments, synthesized the primers listed in Table 2.

The 5'-terminal and the 3'-terminal sequences of this strain were obtained, using 5'-RACE and 3'-RACE methods, correspondingly.

As a result, the full genome sequence of the modified virus was found to consist of 7427 nucleotides, excluding the poly a sequence (Appendix 3). The untranslatable 5'-terminal (5'NTR) contains 742 nucleotides, followed by coding part, starting with start codon (AUG) at position 743, containing codons for 2194 amino acids and ending with stop codon (UAA) at position 7325 (Appendix 3). The untranslatable 3'-terminal (3'NTR) of this strain contains 100 nucleotides, followed by poly A sequence.

### Example 3.4. Comparison of genomes of modified virus (MV) and native strain

Comparison of genomes of modified virus (MV) and starting strain is provided in Appendix 4.

The difference in nucleotide sequence, calculated by programme Vector NTI is substantial, 10% for the complete genome and 12% for the part coding the virus coat proteins. The amino acid sequences for the modified and starting strains are listed in Appendix 5.

### Example 3.5. The genome sequence of the modified virus after propagation for 12 months

The changes in the sequence of modified virus (MV) genome after continuous passaging for 12 months did not exceed 0.7% of the initial sequence.

All found changes were one nucleotide replacements, partially the mute mutations (without change of amino acid). If the amino acid was changed, its position was in the genome polymorphic part, evidently without relevant influence on virus activity.

### Example 4. Virus passaging

Virus MV was passaged by known methods and propagated for 12 months in human embryonal lung culture MRC 5 (Instituto Zooprofilattico Sperimentale della Lombardia e dell Emilia, Brescia - Laboratorio Centro Substrati Cellulari, Catalogue No. BS CL 68 (origin: American Type Culture centre Collection, Rockville, Md, USA), free of bacteria, viruses, fungi or mycoplasmas, and later stored frozen at -70 °C.

### Example 5. Determination of anti-cancer activity of the modified virus (MV)

In experiments with cell lines, MV was found to cytotoxic for melanoma cell lines FM9, FM55, FM94 and SK-Mel26, gastric carcinoma cells, human oral squamous cell carcinoma SCC25 cells, human epithelial cell line derived from a lung carcinoma (A549), acute monocyte leukemia THP-1 cells, rabdomiosarcoma RD cells, human pancreatic adenocarcinoma HPAF-II cells, human breast adenocarcinoma cells (MCF-7) as well as on primary cell cultures of gastric adenocarcinoma GC1 and thyroid cancer line HA007.

Thus, for example, MV injections for 3 days caused reducing of sarcoma M-1 mass in 55% (in 11 of 22) of animals, compared with 6% (in 1 of 18) spontaneous regression in the control group.

Transplanting sarcoma KRS-321 on Day 5 after the injecting MV in a dose 15x10⁶ TCID₅₀ on Wistar rats in 44% of animals (11/25) the regression of tumour was observed, while in the control group there were no cases of regression.

Testing the anti-cancer activity of the virus sample after the 12 months passaging on the same cancer cell lines and transplanted tumours no statistically significant difference from the original MV was observed.

Neither MV nor the virus passaged for 12 months caused any toxic reactions in intact mice.

### Example 6. Anti-cancer activity of modified virus in treating patients

Treating of melanoma patients by the modified virus (MV) was conducted according to the following scheme: therapy was commenced 2-3 weeks after the excision of the tumour by intramuscular administration of 2 ml of solution with titer 2×10⁶ TCID₅₀/ml - 2×10⁸ TCID₅₀/ml for 3 days consecutively with supporting injections at monthly intervals according to the same 3 day schedule. After the fourth month, the virus preparation was administered once monthly for the next 8 months. In the next 2 years the supporting therapy was continued with the same dose, gradually increasing the interval between administrations to 6, 8 and 12 weeks.

In a clinical pilot study, a group of 46 melanoma stage I patients no progress of melanoma was observed for 50 months in 43 patients, treated with MV. In the control group, melanoma progressed for 10 of 31 patients undergoing standard therapy.

In a 50 months study of 44 stage II melanoma patients the progress of melanoma was stopped in 38 patients, compared to control group of 36 patients undergoing standard therapy, where melanoma did not progress in 15 patients, but did progress in 21 patients.

The efficiency of treatment is characterized by the following examples:
Case 1. Female, age 76 , *Melanoma cutis dorsi*
   Op. 11.09.2009. *Excisio tu cutis dorsi*
   pT4b N0 M0
   SN biopsy was not performed
   *Ex consilio:* follow-up
   Op. 07.04.2010. *LAE axillaris sin.*
   *Mts I*/*n axillaris sin*
   *Ex consilio: Roferon*
   *Roferon* 6 mil 3x per week from 24.06.2010 till 30.08.2010.
   The treatment was discontinued due to the side effects.
   From October 2010 the therapy with virus preparation in 2 ml dose with titer *2*×1*0⁶ TCID₅₀*/*ml - 2*×*10⁸ TCID₅₀*/*ml* was commenced. The treatment was well tolerated, and no progression of the disease was documented until 01.02.2012.
Case 2. Female, age 42 , *Melanoma cutis dorsi*
   Op. 25.05.2008. *Excisio tu cutis dorsi*
   pT4a N0 M0, *Clark* V, *Breslow* 9 mm
   SN biopsy was not performed
   Virus preparation (2 ml with titer *2*×*10⁶ TCID₅₀*/*ml - 2*×*10⁸ TCID₅₀*/*ml was administered* from 27.06.2008 till 27.06.2011.
   21.01.2011. US examination: recurrence in the scar
   Op. 02.02.2011. *Excisio.* Histological examination: granuloma.
   Virus preparation (2 ml with titer *2*×*10⁶ TCID₅₀*/*ml - 2*×*10⁸ TCID₅₀*/*ml was continued* till 27.06.2011.
   During the observation period (till December 2011) no evidence of the disease progression was documented.
Case 3. Female, age 57 , *Melanoma cutis dorsi*
   Op.19.08.2007. *Excisio tu cutis dorsi*
   P T3b N0 M0
   SN biopsy was not performed
   Recommendations: follow-up
   Op. 10.12.2009. *LAE colli dx.* Histological examination: *mts l*/*n colli dx* Progression of the disease - US examination on 22.02.2010: *mts l*/*n colli* 22.02.2010. *Ex consilio:* no surgery was recommended due to bulky disease Virus preparation (2 ml with titer *2*×*10⁶ TCID₅₀*/*ml - 2×10⁸ TCID₅₀*/*ml was administered* from 22.02.2010 and still is in progress.
   Last visit at clinic on 22.11.2011 - the disease has stabilized.
Case 4. Female, age 58 , *Melanoma cutis dorsi*
   Op. April 2004. *Excisio tu cutis dorsi, LAE axillaris sin.*
   pT4b, N2c, M0 (*Breslow* 15 mm)
   *Reexcisio* January 2006, September 2006 (local recurrence)
   Therapy with IFN from October 2006 till May 2007.
   *Reexcisio cum dermoplasticum* February 2007, May 2007, September 2007. Virus preparation (2 ml with titer *2*×*10⁶ TCID₅₀*/*ml - 2*×*10⁸ TCID₅₀*/*ml was administrated from February 2008 till April* 2011.
   Visceral metastasis February 2011.
   *Exitus letalis* October 2011.

### Dose form and administration

The viral preparation for therapeutic treatment can be in the form of injectable aqueous solution containing the modified virus having the stable genome sequence as explained above, for example in the titer of 2×10⁶ TCID₅₀/ml - 2×10⁸ TCID₅₀/ml. The solution carrying the virus can be any physiologically acceptable sterile solution, especially sodium chloride solution. The preparation is stored and transported in frozen condition and defrozen at room temperature before the use. The preparation can be in vials or other container units in volumes that correspond a single dose injected at a time to the patient.

The preparation can be administered by injecting it intramuscularly (*i.m.*) to the patient after the excision of the tumour in question, when the wouknd has healed. The dosage can be 2 ml of the above-mentioned solution at a time The intramuscular administration by injection is repeated according to the planned therapy schedule
Appendix 1: **Seq ID No 1**
   **Sequence of the modified virus** 
0 UUAAAACAGC CUGUGGGUUG UUCCCACCCA CAGGGCCCAC UGGGCGCUAG
50 CACACUGGUA UCACGGUACC CUUGUGCGCC UGUUUUAUCU UCCCCUCCCC
100 ACUGUAACUU AGAAGAAUGA CAUAAACGGU CAACAGAUAG CUCAGUACAC
150 CAACUGAGCC CCGACCAAGC ACUUCUGUUA CCCCGGACCG AGUAACAAUA
200 GGCUGCUCGC GCGGCUGAAG GUGAAAACGU UCGUUACCCG GCCAAUUACU
250 UCGAGAAACC UAGUACCACC AUGAAGGUUG CGCAGCGUUU CGCUCCGCAC
300 AACCCCAGUG UAGAUCAGGU CGAUGAGUCA CCGCACUCCC CACGGGCGAC
350 CGUGGCGGUG GCUGCGCUGG CGGCCUGCCU AUGGGGCAAC CCAUGGGACG
400 CUUCAAUACU GACAUGGUGC GAAGAGUCUA UUGAGCUAAU UGGUAGUCCU
450 CCGGCCCCUG AAUGCGGCUA AUCCUAACUG CGGGGCAAGU GCCCACAAAC
500 CAGUGGGUGG CUUGUCGUAA CGGGUAACCC UGCAGCGGAA CCGACUACUU
550 UGGGUGUCCG UGUUUCCUUU UAUUCUUAUU CUGGCUGCUU AUGGUGACAA
600 UUGAGAGAUU GUURCCAUAU AGCUAUUGGA UUGGCCRUCU GGUGAGUAAC
650 AGAGCAAUCA UAUUCCUCUU UGUUGGAUUU AUACCACUUG AUUCCACUAG
700 UUACAACACU CUGCUACAUA UUAUUUGCUU AAAUACAAGA AGAUGGGAGC
750 ACAAGUAUCG ACACAAAAGA CUGGUGCACA CGAGACCSGU UUGAGCGCUA
800 ACGGACACUC UAUCAUUCAC UAUACCAACA UCAACUACUA CAAAGAUGCA
850 GCAUCCAACU CAGCCAACAG GCAGGAUUUC ACCCAGGAUC CAGGUAAGUU
900 CACUGAACCG GUCAAGGAUA UCAUGAUCAA AUCGAUGCCC GCCCUAAACU
950 CACCGUCCGC GGAGGAGUGC GGGUACAGCG ACAGGGUGAG AUCCCUAACG
1000 CUCGGCAACU CAACCAUUAC CACUCAAGAA AGUGCAAACG UAGUUGUUGG
1050 CUAUGGCAGG UGGCCAGAGU ACUUGAAAGA UGAAGAAGCU ACUGCGGAAG
1100 AUCAGCCAAC ACAACCCGAU GUAGCCACRU GCAGGUUCUA CACGUUGGAA
1150 UCCGUCCAGU GGGAGAAAAA UAGCGCUGGA UGGUGGUGGA AGUUCCCCGA
1200 AGCACUUAAG GACAUGGGCC UCUUUGGUCA GAACAUGCUU UACCACUAUC
1250 UCGGUAGAGC AGGCUACACU AUACAUGUGC AGUGCAACGC AUCCAAAUUU
1300 CAUCAGGGCU GUCUACUUGU UGUCUGUGUA CCUGAAGCUG AGAUGGGGUG
1350 UUCCCAGACG GACAAAGAGG UUGCUGCGAU GAACCUCACG AAGGGUGAAA
1400 CGGCGCACAA GUUUGAACCA ACCAAAACCA CAGGCGGCCA CACAGUGCAA
1450 UCCAUAGUGU GCAACGCGGG UAUGGGCAUC GGCGUGGGGA ACCUCACCAU
1500 CUACCCUCAC CAGUGGAUCA ACUUGCGCAC UAAUAACUGC GCUACAAUUG
1550 UGAUGCCGUA UAUAAAUUCA GUACCCAUGG AUAACAUGUU UAGGCACUAC
1600 AAUUUCACGC UAAUGGUGAU CCCAUUUGCA CCCCUGGAUU ACAAUGCCCA
1650 AGCAUCUGAG UACGUACCUG UAACUGUCAC AAUAGCCCCA AUGUGUGCAG
1700 AAUACAAUGG UUUAAGGCUG GCUUACCAGC AAGGGCUGCC AGUGCUAAAU
1750 ACACCGGGAA GCAAUCAGUU UAUGACAUCG GAUGAUUUUC AAUCCCCUUC
1800 GGCUAUGCCA CAAUUUGAUG UGACUCCGCA CAUGGACAUC CCAGGUGAAG
1850 UGCACAACCU CAUGGAGAUU GCAGAAGUUG AUUCGGUGGU ACCUGUUAAC
1900 AACACUGCGG CCAAUCUGCA AAGCAUGGAC GCAUAUCACA UAGAGGUGAA
1950 CRCAGGAAAU CACCAAGGUG AAAAGAUAUU CGCUUUCCAG AUACAACCCG
2000 GGCUGGAUUC AGUGUUUAAG AGAACACUGC UAGGUGAAGU GCUCAAUUAU
2050 UACGCGCACU GGUCAGGGAG CAUUAAGCUA ACAUUCACAU UUUGUGGUUC
2100 AGCAAUGGCC ACGGGCAAGC UACUCUUAGC AUACUCCCCA CCUGGCGCCG
2150 AUGUACCGGC UAGCAGAAAG CAGGCAAUGA UGGGAACCCA UAUCAUCUGG
2200 GACUUAGGGC UGCAAUCCAG UUGCGUUCUA UGUAUUCCAU GGAUCAGUCA
2250 GACACAUUAU CGCCUAGUGC AACAGGAUGA GUACACCAGC GCCGGCAAUG
2300 UCACCUGCUG GUAUCAGACA GGUAUAGUGG UUCCACCCGG CACACCCAAC
2350 AAGUGUGUCG UCCUGUGCUU UGUGUCAGCG UGUAAUGACU UCUCCGUGCG
2400 CAUGCUGCGU GACACACCAU UCAUCGGCCA AACAACACUG CUACAAGGUG
 
2450 AUACGGACGU GGCCGUCAAC AAUGCAGUAG CCAGGGUAGC UGAUACAAUU
2500 GCCAGUGGGC CCAGCAACUC CACUAGCAUU CCUGCACUAA CCGCAGUUGA
2550 GACUGGGCAC ACAUCACAGG UAGAGCCUAG UGAUACAAUG CAAACACGGC
2600 AUGUAAAGAA CUACCAUUCG CGAUCUGAAU CAACAAUAGA GAACUUCCUU
2650 AGCCGGUCGG CCUGUGUAUA UAUUGAAGAG UACUUUACCA AAGAUCAAGA
2700 CAGCGCCAAU AGGUACAUGU CAUGGACUAU AAAUGCUAGA AGGAUGGUGC
2750 AAUUGAGGCG AAAGUUUGAA CUGUUCACAU ACAUGCGGUU UGAUAUGGAG
2800 AUCACAUUUG UUAUCACUAG UAGACAACUG CCUGGGACUA GCAUCGCGCA
2850 AGACAUGCCG CCACUGACAC ACCAAAUCAU GUAUAUACCC CCUGGUGGUC
2900 CAGUACCAAA CAGUGUGACC GAUUUUGCAU GGCAAACUUC GACUAAUCCA
2950 AGUAUCUUUU GGACUGAGGG CAAUGCCCCC CCGCGUAUGU CCAUACCAUU
3000 UAUAAGCAUA GGGAAUGCAU ACAGCAACUU UUAUGACGGR UGGUCGCACU
3050 UCUCACAAAA UGGGGUAUAC GGCUACAAUG CAUUAAACAA CAUGGGCAAA
3100 UUAUACGCAC GCCAUGUGAA CAAAGACACA CCGUACCAGA UGUCCAGUAC
3150 GAUUCGUGUG UACUUUAAAC CCAAACAUAU CAGAGUGUGG GUGCCAAGAC
3200 CACCACGUUU GUGCCCCUAU AUUAAAUCUA GUAACGUUAA CUUUGACCCA
3250 ACCAACCUAA CUGAUUCAAG AUCAAGUAUA ACAUAUGUGC CAGACACUAU
3300 CCGUCCGGAA GUCCGUACAG CUGGAAAAUU CGGCCACCAG UCCGGUGCUG
3350 UUUACGUGGG UAAUUACAGA AUAGUGAACA GGCACCUCGC CACGCACAAC
3400 GACUGGCAAA ACUGUGUGUG GGAAGACUAC AACAGAGACC UCCUUGUGAG
3450 CACCACUACA GCCCAUGGGU GUGACACUAU AGCCAGAUGU CAGUGCACAG
3500 CAGGCGUAUA UUUUUGUGCC UCAAGGAACA AACAUUACCC AGUCACCUUC
3550 GAGGGGCCAG GCUUGGUGGA AGUUCAGGAG AGCGAGUACU ACCCAAAAAG
3600 AYAUCAGUCC CACGUGCUUC UAGCUGCAGG AUUUUCUGAA CCGGGCGAUU
3650 GUGGCGGAAU CCUCAGAUGU CAACACGGCG UGAUCGGUAU CGUCACCAUG
3700 GGUGGAGAGG GGGUCGUUGG GUUUGCCGAC GUCAGAGACC UACUGUGGUU
3750 AGAGGAUGAU GCCAUGGAAC AGGGCGUAAG AGACUAUGUU GAACAACUAG
3800 GAAAUGCUUU CGGCUCAGGU UUCACCAAUC AAAUUUGUGA ACAGGUCAAC
3850 CUCCUCAAAG AGUCAUUGGU UGGACAGGAU UCUAUUCUGG AAAAAUCCCU
3900 UAAGGCUCUA GUUAAGAUUA UCUCAGCACU GGUCRUUGUA GUGAGAAAUC
3950 ACGAUGAUCU CAUAACGGUU ACCGCCACUC UAGCUUUAAU UGGUUGCACC
4000 UCUUCUCCGU GGCGGUGGCU CAAGCAGAAG GUGUCACAAU AUUAUGGAAU
4050 ACCCAGGGCC GAGCGACAAA ACAAUAGCUG GCUCAAGAAG UUUACUGAGA
4100 UGACCAACGC CUGCAAGGGC AUGGAGUGGA UAGCCAUAAA AAUUCAAAAG
4150 UUUAUUGAGU GGCUUAAAGU CAAGAUUCUG CCGGAAGUGA AGGAAAAACA
4200 CGAGUUCCUC AACAGGCUAA AGCAAUUACC ACUCCUAGAG AGCCAGAUUG
4250 CAACCAUAGA GCAGAGUGCA CCAUCGCAGA GUGAUCAAGA GCAACUCUUC
4300 UCCAACGUCC AGUACUUCGC CCAUUAUUGC AGAAAGUAUG CGCCAUUGUA
4350 CGCUGCCGAA GCGAAGAGAG UGUUCUCACU UGAGAAGAAA AUGAGCAACU
4400 ACAUACAGUU CAAGUCCAAA UGCCGUAUUG AGCCUGUAUG CUUACUCCUA
4450 CAUGGCAGCC CAGGGGCCGG AAAGUCCGUG GCCACCAACU UGAUUGGCAG
4500 AUCCCUCGCA GAAAAACUCA ACAGCUCUGU RUACUCCCUA CCACCAGACC
4550 CCGACCACUU UGACGGCUAC AAGCAGCAAG CGGUCGUGAU CAUGGAUGAC
4600 UUAUGCCAAA AUCCUGAUGG AAAAGAUGUC UCACUAUUUU GUCAGAUGGU
4650 UUCUAGCGUG GACUUUGUAC CACCGAUGGC UGCGCUAGAG GAAAAAGGAA
4700 UCCUAUUUAC CUCCCCGUUC GUGUUGGCAU CAACCAACGC UGGGUCCAUC
4750 AAUGCACCCA CUGUGUCUGA CAGCAGAGCG CUCGCUAGGA GAUUCCACUU
4800 UGACAUGAAC AUUGAAGUCA UUUCUAUGUA CAGUCAAAAC GGCAAGAUCA
4850 ACAUGCCCAU GUCAGUUAAA ACAUGUGAUG AAGAGUGUUG UCCAGUUAAC
4900 UUCAAAAGGU GCUGCCCGUU GGUGUGUGGA AAGGCYAUGC AAUUCAUUGA
4950 UAGGAGAACU CAAGUUAGAU AUUCGCUGGA CAUGCUAGUU ACUGAAAUGU
5000 UUAGGGAGUA UAACCAUAGA CACAGUGUGG GAGCCACUCU UGAAGCUCUG
5050 UUCCAAGGGC CACCAGUCUA CAGAGAGAUC AAAAUCAGCG UCGCCCCAGA
 
5100 GACACCCCCA CCACCAGCUA UUGCUGAUUU ACUGAAAUCA GUGGACAGUG
5150 AAGCUGUGAG GGAAUACUGC AAGGAGAGAG GGUGGCUUGU GCCAGAGAUC
5200 AAUUCUACCC UACAAAUAGA GAAGCAUGUG AGUAGAGCAU UCAUAUGUUU
5250 ACAAGCCCUA ACCACGUUUG UUUCAGUUGC UGGUAUAAUA UACAUUAUUU
5300 ACAAAUUAUU UGCAGGUUUC CAAGGCGCCU ACACAGGGAU GCCCAACCAG
5350 AAACCUAAGG UGCCCACCCU GAGACAGGCC AAAGUACAGG GCCCAGCGUU
5400 UGAGUUCGCU GUGGCGAUGA UGAAAAGGAA CGCCAGUACA GUAAAAACCG
5450 AGUACGGUGA AUUCACCAUG CUUGGCAUUU ACGACAAGUG GGCGGUGUUA
5500 CCGCGCCACG CCAAGCCUGG CCCCACCAUC UUGAUGAAUG AUCAGGAAGU
5550 CGGCGUGUUG GAUGCCAAGG AACUAGUUGA UAAAGAUGGG ACAAAUCUAG
5600 AAUUGACUCU CCUGAAGCUC AACCGUAACG AAAAGUUCAG AGAUAUUAGG
5650 GGGUUUCUAG CAAGAGAAGA GGUUGAAGUG AAUGAAGCUG UCCUAGCAAU
5700 AAAUACAAGC AAAUUCCCUA ACAUGUACAU ACCAGUGGGC CAGGUGACUG
5750 ACUACGGGUU UCUGAACCUG GGAGGGACUC CCACGAAGAG AAUGCUCAUG
5800 UAUAACUUCC CAACUAGAGC AGGUCAGUGU GGAGGUGUCC UCAUGUCAAC
5850 AGGGAAAGUC CUGGGAAUAC AUGUAGGAGG GAAUGGACAU CAAGGGUUCU
5900 CAGCGGCACU CCUCAGGCAC UACUUCAACG AGGAGCAGGG UGAAAUAGAA
5950 UUCAUUGAGA GCUCAAAGGA CGCGGGAUUC CCUGUGAUCA ACACUCCCAG
6000 UAAGACAAAA UUGGAACCAA GUGUGUUUCA CCAGGUGUUC GAGGGCAACA
6050 AGGAACCAGC GGUCCUUAGA AAUGGGGACC CACGACUCAA AGCCAACUUC
6100 GAGGAAGCAA UCUUCUCCAA GUACAUUGGC AAUGUCAACA CGCAUGUAGA
6150 UGAGUACAUG UUGGAGGCUG UGGACCAUUA UGCAGGACAA CUAGCUACUC
6200 UGGACAUCAG UACGGAGCCC AUGAAGCUAG AGGACGCCGU GUAUGGUACA
6250 GAGGGGCUGG AAGCACUAGA CCUAACCACC AGUGCAGGCU ACCCUUACGU
6300 GGCCCUGGGC AUCAAGAAAA GAGAUAUUCU AUCUAAGAAG ACUAAAGACC
6350 UCACUAAGUU GAAGGAAUGC AUGGACAAAU AUGGCCUAAA UUUGCCAAUG
6400 GUAACCUACG UCAAAGAUGA GUUGAGAUCU GCUGAGAAGG UGGCCAAGGG
6450 AAAAUCCAGG CUUAUUGAGG CUUCUAGUCU CAAUGACUCA GUAGCAAUGA
6500 GGCAAACAUU UGGAAAUUUA UAUAAGACCU UUCACCUCAA CCCGGGCAUC
6550 GUUACGGGCA GUGCUGUUGG GUGUGAUCCA GAUGUGUUUU GGAGCAAGAU
6600 CCCUGUUAUG CUUGAUGGAC AUCUCAUAGC UUUUGACUAU UCAGGCUAUG
6650 ACGCUAGCCU CAGCCCAGUG UGGUUUGCAU GUUUGAAACU UCUCCUAGAG
6700 AAACUAGGGU AUACAAACAA GGAAACAAAC UACAUAGAUU ACCUCUGUAA
6750 UUCCCAUCAC CUGUAUAGAG ACAAGCACUA CUUUGUAAGA GGCGGUAUGC
6800 CAUCAGGGUG UUCAGGCACC AGCAUAUUUA AUUCCAUGAU UAACAACAUC
6850 AUAAUCAGGA CUCUCAUGCU GAAGGUUUAU AAAGGCAUUG AUUUGGACCA
6900 AUUCAGAAUG AUUGCCUAUG GGGAUGAUGU GAUUGCUUCC UAUCCGUGGC
6950 CUAUCGAUGC UUCGCUGUUA GCUGAAGCAG GAAAAGAUUA UGGUUUAAUC
7000 AUGACCCCAG CAGACAAAGG CGAGUGCUUC AACGAGGUAA CCUGGACGAA
7050 UGUGACCUUU CUGAAAAGGU ACUUUAGGGC AGAUGAGCAA UACCCAUUUC
7100 UGGUCCAUCC UGUUAUGCCA AUGAAGGACA UCCAUGAGUC UAUUAGGUGG
7150 ACCAAAGAUC CCAAGAACAC ACAGGAUCAU GUGCGCUCGC UGUGCCUAUU
7200 GGCUUGGCAC AACGGGGAGC AAGAAUAUGA GGAGUUUAUU CGCAAGAUCA
7250 GAAGCGUGCC CGUUGGGCGC UGCUUGACCC UACCCGCUUU UUCAACACUG
7300 CGCAGGAAGU GGCUGGACUC CUUUUAAAAU UAGAGCAUAA UUAGUAAAUC
7350 AUAAUUGGCU UAACCCUACC GCAUGAACCG AACUUGAUAA AAGUGCGGUA
7400 GGGGUAAAUU CUCCGCAUUC GGUGCGG
Appendix 2: **Seq ID No 2**
   **Sequence of the unmodified (native) virus** 
0 TTAAAACAGC CTGTGGGTTG TTCCCACCCA CAGGGCCCAC TGGGCGCTAG
50 CACACTGGTA TCACGGTACC TTTGTGCGCC TGTTTTATAT CCCCCTCCCC
100 ACTGTAACTT AGAGAAATCA CATAAACGAT CAATAGAAGG CGCAGCACAC
150 CAGCTGAGTC TTGACCAAGC ACTTCTGTTT CCCCGGACTG AGTATCAATA
200 GACTGCTCAC GCGGTTGAAG GAGAAAACGT TCGTTACCCG GCCAACTACT
250 TCGAGAAACC TAGTACCACC ATGAAAGTTG CGCAGTGTTT CGCTCAGCAC
300 AACCCCAGTG TAGATCAGGT CGATGAGTCA CCGCATTCCC CACGGGCGAC
350 CGTGGCGGTG GCTGCGTTGG CGGCCTGCCT ATGGGGCAAC CCATGGGACG
400 CTTCAATACT GACATGGTGC GAAGAGTCTA TTGAGCTAGT TGGTAGTCCT
450 CCGGCCCCTG AATGCGGCTA ATCCTAACTG CGGAGCAAGT GCCCACAAAC
500 CAGTGGGTAG CTTGTCGTAA CGGGCAACTC TGCAGCGGAA CCGACTACTT
550 TGGGTGTCCG TGTTTCCTTT TATTCTTATT CTGGCTGCTT ATGGTGACAA
600 TTGAGAGATT GTTACCATAT AGCTATTGGA TTGGCCATCC GGTGACTAAC
650 AGAGCAATTA TATACCTCTT TGTTGGATTT ATACCACTTA ATTCCACTAA
700 TTACAACACT CTGCTACACA TTATTTACTT AAAACCAAGA AGATGGGAGC
750 ACAAGTATCA ACACAAAAAA CTGGTGCACA TGAGACCSGT TTGAGCGCTA
800 ACGGAAGCTC CATCATTCAC TACACCAACA TCAATTACTA CAAAGATGCA
850 GCATCCAACT CAGCCAACAG GCAAGACTTC ACCCAAGATC CAGGCAAATT
900 CACCGAACCG GTCAAGGATA TCATGATCAA GTCAATGCCC GCCCTAAACT
950 CACCGACCGT GGAGGAGTGT GGGTACAGTG ATAGGGTGAG ATCCATAACG
1000 CTCGGCAACT CAACCATTAC CACTCAGGAG AGTGCAAATG TAGTTGTTGG
1050 CTATGGCGGG TGGCCAGAGT ACTTGAAAGA TGAAGAAGCT ACTGCGGAAG
1100 ATCAACCAAC ACAACCCGAT GTAGCCACAT GCAGGTTTTA CACGCTGGAA
1150 TCCGTCCAGT GGGAGAAAAA TTCCGCTGGA TGGTGGTGGA AGTTCCCCGA
1200 AGCACTTAAG GACATGGGCC TCTTTGGTCA AAACATGCAT TACCACTACC
1250 TCGGTAGAGC AGGCTACACT ATACACGTGC AGTGCAATGC ATCCAAATTC
1300 CACCAAGGCT GTCTACTTGT TGTCTGTGTA CCTGAGGCTG AGATGGGGTG
1350 TTCCAAAGTG GACGGTACTG TAAATGAGCA GGAATTGACG GAGGGTGAAA
1400 CGGATATGAA GCTTGAACCC ACCAGAACCA CAGGCGTACG CCGAGTGCAA
1450 TCCGCAGTGT ACAACGCGGG TATGGGCGTC GGCGTGGGGA ACCTCACCAT
1500 CTTCCCTCAC CAGTGGATCA ACCTGCGCAC TAACAACTGT GCTACAATTG
1550 TGATGCCATA CATAAATAGT GTACCCATGG ATAACATGTT TAGGCACTAC
1600 AACTTCACGC TAATGATGAT CCCATTTGCA CCCCTGGATT ACACCAACCA
1650 AGCATCTACG TACGTACCTA TAACTGTCAC AATAGCACCA ATGTGTGCTG
1700 AATACAATGG TTTGAGGCTC GTTACCTCGC AAGGGTTGCC AGTGATGAAC
1750 ACACCGGGAA GCAATCAGTT CCTGACATCG GATGACTTTC AATCACCTTC
1800 GGCTATGCCA CAATTTGATG TGACTCCAGA CATGGACATC CCAGGTGAAG
1850 TGAACAACCT CATGGAGATT GCAGAGGTTG ACTCGGTGGT ACCTGTTAAC
1900 AACAATGAGG CCAATCTGAA AAGCATGGAC GCATACCGCA TACCGGTGAA
1950 CRCAGGAAAT CAACAAGGTG AAAAGATATT TGGTTTCCAA ATACAACCCG
2000 GGCTTGATTC AGTGTTTAAG AGAACACTGC TAGGTGAGAT GCTCAATTAT
2050 TACACGCACT GGTCAGGGAG CATTAAGCTA ACATTTATGT TTTGTGGTTC
2100 AGCAATGGCC ACGGGCAAAT TACTCTTAGC ATACTCACCA CCTGGCGCCG
2150 ATGTACCGAC TAGCAGAAAG GAGGCAATGC TGGGAACCCA TGTCATCTGG
2200 GACTTTGGGC TGCAATCCAG TTGTGTTCTG TGTGTTCCAT GGATCAGCCA
2250 GACACACTAC AGGTTGGTGC AGCAGGATGA GTACACCGGC GCCGGCTATA
2300 TCACCTGCTG GTACCAAACA AGTATAGTGG TTCCACCCGG CACACCCAAA
2350 AAGTGTGTCA TCCTGTGCTT TGTGTCAGCG TGTAATGATT TCTCCGTGAG
2400 CATGCTGAGT GACACACCAT TCATCGGCCA AACAGCACTG CTGCAGAGCC
2450 CTGTGGAAGA AGCTGAAGAG AACGCAGTTG CACGTGTGGC TGACACAATT
2500 GCCAGTGGGC CCAGCAACTC CGAGAGCGTT CCTGCACTAA CAGCAGTTGA
2550 GACTGGGCAC ACATCACAGG TAGTGCCTAG TGACACAATG CAAACAAGGC
2600 ATGTGAAGAA CTACCATTCG AGATCTGAGT CAACAATAGA GAACTTCCTT
2650 AGCAGGTCCG CCTGTGTGTA TATTGAAGAG TACTATACCA ACACTGAAAC
2700 CAGACAAAAT TTATACATGT TGCCCACTAT AAATACTAGA TGGATGGTGC
2750 AATTGAGGAG AAAGTTTGAG ATGTTCACAT ACATGAGGTT TGACATGGAA
2800 ATCACATTTG TTATCACTAG TAGACAACTG CATCGAACTA GCATGCCGCA
2850 GGACATGCCG GTACTGACAC ACCAAATCAT GTATGTACCA CCTGGTGGTC
 
2900 CAGTACCAAA CAGTGTGGAC GATTACGCAT GGCAAACTTC GACTAACCCA
2950 AGTGTCTTTT GGACTGAGGG CAATGCCCCA CCGCGTATGT CCATACCATT
3000 CATAAGCATA GGGAATGCAT ACAGCAACTT TTATGATGGG TCCTCGCACT
3050 TCTTACAATA TGGGGTATAT GGCTACAACA CATTAAACAA CATGGGGAAA
3100 TTATACGTAC GCCATGTGAA CAACCACACA CCATACCAAA TGACCAGTAC
3150 GGTTAGTGTG TACTTTAAAC CCAAACATGT CAGAGCGTGG GTGCCGAGAC
3200 CACCACGTCT GTGCCCCTAC AAAAATGCAT GGAACGTTAA CTTTGAACCA
3250 ACAAACGTAA CTGATTCAAG ATCAAGTATC ACATATATTC CTGAGACGGT
3300 CAAACCAGAC CTATCAAAAG CTGGAGCTTT CGGCCACCAG TCCGGTGCTG
3350 TTTATGTGGG TAACTACAGA GTGGTGAATA GGCACCTCGC CACGCACAAC
3400 GACTGGCAAA ACTGTGTGTG GGAAGACTAC AACAGAGACC TCCTTGTGAG
3450 CACCACCACA GCCCATGGGT GTGACACCAT AGCCAGATGC CAGTGCACAA
3500 CAGGCGTGTA CTTTTGTGCC TCAAGGAACA AACACTACCC AGTCACCTTT
3550 GAGGGGCCAG GCCTGGTGGA AGTTCAGGAG AGTGAGTACT ACCCAAAAAG
3600 ATACCAATCC CATGTGCTTC TAGCTGCAGG ATTTTCTGAA CCAGGCGATT
3650 GTGGTGGAAT CCTCAGGTGT GAACATGGTG TCATCGGTAT CGTCACCATG
3700 GGTGGAGAGG GGGTCGTTGG GTTTGCCGAC GTCCGAGACC TACTGTGGTT
3750 AGAGGATGAT GCCATGGAAC AGGGCGTAAG AGACTATGTT GAACAACTAG
3800 GAAATGCTTT TGGCTCAGGT TTCACCAACC AAATTTGTGA ACAAGTCAAC
3850 CTCCTCAAAG AGTCACTGGT TGGACAGGAC TCCATTCTGG AGAAATCCCT
3900 TAAAGCCCTA GTTAAGATTA TCTCAGCACT GGTCATTGTA GTGAGAAATC
3950 ACGATGACCT CATCACAGTG ACTGCCACTC TAGCCCTCAT TGGTTGCACC
4000 TCTTCTCCAT GGCGGTGGCT CAAACAGAAA GTGTCACAAT ATTATGGAAT
4050 ACCCATGGCT GAGCGACAAA ACAATGGCTG GCTCAAGAAG TTCACTGAGA
4100 TGACCAATGC CTGCAAGGGC ATGGAGTGGA TAGCCATCAA AATTCAAAAA
4150 TTTATTGAGT GGCTTAAAGT CAAGATCTAC CAGAAGTGTA GGAAAAACAT
4200 GAGTTCCTCA ACAGACTATA ACAACTACCA CTCTTGGAAG AGTCAGATTG
4250 CCACCATAGA ACAAAGTGCA CCATCGCAGA GTGACCAGGA GCAACTGTTT
4300 TCCAATGTCC AGTACTTCGC CCACTATTGC AGAAAGTATG CGCCACTGTA
4350 TGCAGCTGAG GCAAAGAGAG TGTTCTCCCT TGAGAAGAAA ATGAGCAATT
4400 ACATACAGTT CAAGTCCAAA TGCCGTATTG AGCCTGTATG TTTGCTCNTA
4450 CATGGCAGCC CAGGGGCCGG AAAATCCGTG GCCACCAACC TGATTGGCAG
4500 ATCACTCGCT GAAAAACTCA ACAGCTCAGT GTACTCCCTA CCACCAGACC
4550 CAGATCACTT TGATGGCTAC AAACAGCAAG CGGTCGTGAT CATGGATGAT
4600 CTATGCCAAA ATCCTGATGG AAAAGATGTG TCATTGTTCT GTCAAATGGT
4650 TTCCAGTGTG GACTTTGTAC CACCGATGGC TGCGCTAGAG GAGAAAGGCA
4700 TTCTGTTCAC CTCCCCGTTT GTCCTGGCAT CAACCAATGC TGGGTCCATC
4750 AATGCACCAA CTGTGTCAGA CAGCAGAGCC CTCGCTAGGA GATTCCACTT
4800 TGACATGAAC ATTGAAGTCA TTTCCATGTA CAGTCAAAAT GGCAAGATCA
4850 ACATGCCCAT GTCAGTTAAG ACGTGTGATG AAGAGTGTTG TCCAGTCAAC
4900 TTCAAGAGGT GCTGCCCGCT GGTGTGTGGA AAGGCCATGC AGTTCATTGA
4950 CAGAAGAACT CAAGTTAGAT ACTCGCTGGA CATGCTAGTT ACTGAGATGT
5000 TTAGGGAGTA CAACCACAGA CACAGTGTGG GAGCCACCCT TGAGGCTCTG
5050 TTCCAAGGGC CACCAGTCTA CAGAGAGATC AAAATTAGTG TCGCACCAGA
5100 GACACCACCA CCACCAGCTA TTGCTGACTT ACTGAAATCA GTGGACAGTG
5150 AAGCTGTGAG AGAGTACTGC AAAGAAAAGG GATGGCTTGT GCCAGAGATC
5200 AACTCCACCC TACAAATTGA GAAGCATGTG AGCCGGGCAT TCATCTGTCT
5250 GCAAGCACTA ACCACGTTTG TTTCAGTTGC TGGAATAATA TACATTATTT
5300 ACAAGCTATT TGCAGGTTTC CAAGGCGCAT ACACAGGGAT GCCCAACCAG
5350 AAACCCAAGG TGCCCACCCT GAGACAAGCC AAAGTGCAAG GCCCAGCGTT
5400 TGAGTTTGCT GTGGCGATGA TGAAGAGGAA CTCCAGTACA GTGAAAACCG
5450 AGTACGGTGA GTTCACCATG CTTGGCATTT ATGACAGGTG GGCGGTGTTA
5500 CCACGCCACG CCAAACCTGG CCCAACCATC TTGATGAATG ACCAGGAAGT
5550 CGGCGTGTTG GATGCCAAGG AACTAGTGGA TAAGGATGGG ACAAACCTAG
5600 AACTGACACT CCTGAAGCTC AACAGTAATG AGAAGTTCAG AGACATCAGA
5650 GGGTTCCTAG CCAAAGAAGA GGTTGAGGTG AATGAAGCTG TCCTAGCAAT
5700 AAACACAAGC AAGTTCCCCA ACATGTACAT ACCAGTGGGC CAGGTGACTG
5750 ACTACGGGTT CCTGAACCTG GGTGGGACGC CCACTAAGAG AATGCTCATG
5800 TACAACTTCC CCACTAGAGC AGGTCAGTGT GGTGGTGTCC TCATGTCCAC
5850 TGGGAAAGTC CTGGGGATAC ATGTTGGTGG GAATGGTCAT CAAGGGTTCT
5900 CAGCAGCACT CCTCAAGCAC TACTTCAACG ATGAACAAGG TGAAATAGAG
5950 TTCATTGAGA GCTCAAAGGA CGCGGGGTTC CCTATCATCA ACACACCCAG
6000 CAAGACCAAA CTGGAACCAA GTGTCTTCCA CCAGTGTTTG AAGGCAACAA
6050 AGAACCCAGC AGTCCTCAGA AATGGTGATC CACGACTCAA AGCCAACTTT
6100 GAGGAGGCCA TCTTCTCCAA ATACATTGGC AATGTCAACA CGCATGTGGA
6150 TGAGTACATG TTGGAAGCTG TGGACCATTA TGCAGGACAA CTGGCTACTC
6200 TGGACATCAG CACGGAACCA ATGAAGCTGG AGGATGCCGT GTATGGTACA
6250 GAGGGGCTGG AAGCACTAGA CCTAACAACC AGTGCAGGCT ACCCTTATGT
 
6300 TGCCCTGGGC ATCAAGAAGA GAGACATCCT ATCTAAGAAG ACCAGGGACC
6350 TCACTAAGTT GAAAGAATGC ATGGACAAGT ATGGCCTAAA CCTGCCAATG
6400 GTAACCTATG TGAAAGATGA GCTCAGATCT GCAGAGAAGG TGGCCAAAGG
6450 AAAATCCAGG CTTATTGAAG CTTCCAGTTT GAATGACTCA GTGGCAATGA
6500 GACAGACATT TGGAAACCTG TACAAAACCT TCCACCTCAA CCCAGGCATT
6550 GTGACGGGCA GTGCAGTTGG GTGTGACCCA GATCTGTTTT GGAGCAAGAT
6600 ACCAGTCATG TTGGATGGAC ATCTCATAGC TTTTGATTAC TCAGGCTATG
6650 ATGCTAGCCT CAGCCCAGTG TGGTTTGCAT GTCTGAAACT GCTCCTAGAG
6700 AAGCTTGGGT ACACACACAA GGAAACAAAC TACATAGATT ACCTCTGCAA
6750 CTCCCACCAC CTGTACAGAG ACAAACACTA CTTTGTGCGA GGTGGTATGC
6800 CATCAGGGTG TTCTGGCACC AGCATCTTTA ACTCAATGAT TAACAACATC
6850 ATAATCAGGA CACTCATGCT GAAAGTGTAC AAGGGCATTG ACTTGGACCA
6900 ATTCAGGATT ATTGCCTATG GTGATGATGT GATTGCTTCC TACCCGTGGC
6950 CCATTGATGC TTCCCTGCTA GCTGAAGCAG GAAAAGATTA TGGTTTGATC
7000 ATGACACCAG CAGATAAAGG AGAGTGCTTC AATGAAGTCA ACTGGACGAA
7050 TGTCACCTTC CTGAAAAGGT ACTTTAGAGC AGATGAGCAA TACCCATTCC
7100 TGGTCCACCC TGTTATGCCC ATGAAAGACA TCCATGAATC TATTAGATGG
7150 ACCAAAGATC CAAAGAACAC CCAAGATCAT GTGCGCTCGC TGTGCCTATT
7200 GGCTTGGCAC AATGGGGAGC ACGAATATGA GGAGTTCATT CGCAAAATCA
7250 GAAAGCGTGC CAGTTGGACG CTGTTTGACC CTACCTGCGT TTTCAACCCT
7300 GCGCAGGAAG TGGTTGGACT CCTTTTAAAA TAAAGCACAa TTTAGTAAAT
7350 TTGAATTGGC TTAACCCTAC CGCACTAACC GAACTAGATA ACGGTGCGGT
7400 AGGGGTAAAT TCTCCGCATT CGGTGCGGTC GAGG
Appendix 3: **Seq ID No 3**
   **Sequence of the modified virus after propagation for 12 months** 
0 UUAAAACAGC CUGUGGGUUG UUCCCACCCA CAGGGCCCAC UGGGCGCUAG
50 CACACUGGUA UCACGGUACC CUUGUGCGCC UGUUUUAUCU UCCCCUCCCC
100 ACUGUAACUU AGAAGAAUGA CAUAAACGGU CAACAGAUAG CUCAGUACAC
150 CAACUGAGCC CCGACCAAGC ACUUCUGUUA CCCCGGACCG AGUAACAAUA
200 GGCUGCUCGC GCGGCUGAAG GUGAAAACGU UCGUUACCCG GCCAAUUACU
250 UCGAGAAACC UAGUACCACC AUGAAGGUUG CGCAGCGUUU CGCUCCGCAC
300 AACCCCAGUG UAGAUCAGGU CGAUGAGUCA CCGCACUCCC CACGGGCGAC
350 CGUGGCGGUG GCUGCGCUGG CGGCCUGCCU AUGGGGCAAC CCAUGGGACG
400 CUUCAAUACU GACAUGGUGC GAAGAGUCUA UUGAGCUAAU UGGUAGUCCU
450 CCGGCCCCUG AAUGCGGCUA AUCCUAACUG CGGGGCAAGU GCCCACAAAC
500 CAGUGGGUGG CUUGUCGUAA CGGGUAACCC UGCAGCGGAA CCGACUACUU
550 UGGGUGUCCG UGUUUCCUUU UAUUCUUAUU CUGGCUGCUU AUGGUGACAA
600 UUGAGAGAUU GUUGCCAUAU AGCUAUUGGA UUGGCCAUCU GGUGAGUAAC
650 AGAGCAAUCA UAUUCCUCUU UGUUGGAUUU AUACCACUUG AUUCCACUAG
700 UUACAACACU CUGCUACAUA UUAUUUGCUU AAAUACAAGA AGAUGGGAGC
750 ACAAGUAUCG ACACAAAAGA CUGGUGCACA CGAGACCGGU UUGAGCGCUA
800 ACGGACACUC UAUCAUUCAC UAUACCAACA UCAACUACUA CAAAGAUGCA
850 GCAUCCAACU CAGCCAACAG GCAGGAUUUC ACCCAGGAUC CAGGUAAGUU
900 CACUGAACCG GUCAAGGAUA UCAUGAUCAA AUCGAUGCCC GCCCUAAACU
950 CACCGUCCGC GGAGGAGUGC GGGUACAGCG ACAGGGUGAG AUCCCUAACG
1000 YUCGGCAACU CAACCAUUAC CACUCAAGAA AGUGCAAACG UAGUUGUUGG
1050 CUAUGGCAGG UGGCCAGAGU ACUUGAAAGA UGAAGAAGCU ACUGCGGAAG
1100 AUCAGCCAAC ACAACCCGAU GUAGCCACAU GCAGGUUCUA CACGUUGGAA
1150 UCCGUCCAGU GGGAGAAAAA UAGCGCUGGA UGGUGGUGGA AGUUCCCCGA
1200 AGCACUUAAG GACAUGGGCC UCUUUGGUCA GAACAUGCUU UACCACUAUC
1250 UCGGUAGAGC AGGCUACACU AUACAUGUGC AGUGCAACGC AUCCAAAUUU
1300 CAUCAGGGCU GUCUACUUGU UGUCUGUGUA CCUGAAGCUG AGAUGGGGUG
1350 UUCCCAGACG GACAAAGAGG UUGCUGCGAU GAACCUCACG AAGGGUGAAA
1400 CGGCGCACAA GUUUGAACCA ACCAAAACCA CAGGCGGCCA CACAGUGCAA
1450 UCCAUAGUGU GCAACGCGGG UAUGGGCAUC GGCGUGGGGA ACCUCACCAU
1500 CUACCCUCAC CAGUGGAUCA ACUUGCGCAC UAAUAACUGC GCUACAAUUG
1550 UGAUGCCGUA UAUAAAUUCA GUACCCAUGG AUAACAUGUU UAGGCACUAC
1600 AAUUUCACGC UAAUGGUGAU CCCAUUUGCA CCCCUGGAUU ACAAUGCCCA
1650 AGCAUCUGAG UACGUACCUG UAACUGUCAC AAUAGCCCCA AUGUGUGCAG
1700 AAUACAAUGG UUURAGGCUG GCUUACCAGC AAGGGCUGCC AGUGCUAAAU
1750 ACACCGGGAA GCAAUCAGUU UAUGACAUCG GAUGAUUUUC AAUCCCCUUC
1800 GGCUAUGCCA CAAUUUGAUG UGACUCCGCA CAUGGACAUC CCAGGUGAAG
1850 UGCACAACCU CAUGGAGAUU GCAGAAGUUG AUUCGGUGGU ACCUGUUAAC
1900 AACACUGCGG CCAAUCUGCA AAGCAUGGAC GCAUAUCACA UAGAGGUGAA
1950 CGCAGGAAAU CACCAAGGUG AAAAGAUAUU CGCUUUCCAG AUACAACCCG
2000 GGCUGGAUUC AGUGUUUAAG AGAACACUGC UAGGUGAAGU GCUCAAUUAU
2050 UACGCGCACU GGUCAGGGAG CAUUAAGCUA ACAUUCACAU UUUGUGGUUC
2100 AGCAAUGGCC ACGGGCAAGC UACUCUUAGC AUACUCCCCA CCUGGCGCCG
2150 AUGUACCGGC UAGCAGAAAG CAGGCAAUGM UGGGAACCCA UAUCAUCUGG
2200 GACUUAGGGC UGCAAUCCAG UUGCGUUCUA UGUAUUCCAU GGAUCAGUCA
2250 GACACAUUAU CGCCUAGUGC AACAGGAUGA GUACACCAGC GCCGGCAAUG
 
2300 UCACCUGCUG GUAUCAGACA GGUAUAGUGG UUCCACCCGG CACACCCAAC
2350 AAGUGUGUCG UCCUGUGCUU UGUGUCAGCG UGUAAUGACU UCUCCGUGCG
2400 CAUGCUGCGU GACACACCAU UCAUCGGCCA AACAACACUG CUACAAGGUG
2450 AUACGGACGU GGCCGUCAAC AAUGCAGUAG CCAGGGUAGC UGAUACAAUU
2500 GCCAGUGGGC CCAGCAACUC CACUAGCAUU CCUGCACUAA CCGCAGUUGA
2550 GACUGGGCAC ACAUCACAGG UAGAGCCUAG UGAUACAAUG CAAACACGGC
2600 AUGUAAAGAA CUACCAUUCG CGAUCUGAAU CAACAAUAGA GAACUUCCUU
2650 AGCCGGUCGG CCUGUGUAUA UWUUGAAGAS UACUUUACCA AAGAUCAAGA
2700 CAGCGCCAAU AGGUACAUGU CAUGGACUAU AAAUGCUAGA AGGAUGGUGC
2750 AAUUGAGGCG AAAGUUUGAA CUGUUCACAU ACAUGCGGUU UGAUAUGGAG
2800 AUCACAUUUG UUAUCACUAG UAGACAACUG CCUGGGACUA GCAUCGCGCA
2850 AGACAUGCCG CCACUGACAC ACCAAAUCAU GUAUAUACCC CCUGGUGGUC
2900 CARUACCAAA CAGUGUGACC GAUUUUGCAU GGCAAACUUC GACUAAUCCA
2950 AGUAUCUUUU GGACUGAGGG CAAUGCCCCC CCGCGUAUGU CCAUACCAUU
3000 UAUAAGCAUA GGGAAUGCAU ACAGCAACUU UUAUGACGGA UGGUCGCACU
3050 UCUCACAAAA UGGGGUAUAC GGCUACAAUG CAUUAAACAA CAUGGGCAAA
3100 UUAUACGCAC GCCAUGUGAA CAAAGACACA CCGUACCAGA UGUCCAGUAC
3150 GAUUCGUGUG UACUUUAAAC CCAAACAUAU CAGAGUGUGG GUGCCAAGAC
3200 CACCACGUUU GUGCCCCUAU AUUAAAUCUA GUAACGUUAA CUUUGACCCA
3250 ACCAACCUAA CUGAUUCAAG AUCAAGUAUA ACAUAUGUGC CAGACACUAU
3300 CCGUCCGGAA GUCCGUACAG CUGGAAAAUU CGGCCACCAG UCCGGUGCUG
3350 UUUACGUGGG UAAUUACAGA AUAGUGAACA GGCACCUCGC CACGCACAAC
3400 GACUGGCAAA ACUGUGUGUG GGAAGACUAC AACAGAGACC UCCUUGUGAG
3450 CACCACUACA GCCCAUGGGU GUGACACUAU AGCCAGAUGU CAGUGCACAG
3500 CAGGCGUAUA UUUUUGUGCC UCAAGGAACA AACAUUACCC AGUCACCUUC
3550 GAGGGGCCAG GCUUGGUGGA AGUUCAGGAG AGCGAGUACU ACCCAAAAAG
3600 AUAUCAGUCC CACGUGCUUC UAGCUGCAGG AUUUUCUGAA CCGGGCGAUU
3650 GUGGCGGAAU CCUCAGAUGU CAACACGGCG UGAUCGGUAU CGUCACCAUG
3700 GGUGGAGAGG GGGUCGUUGG GUUUGCCGAC GUCAGAGACC UACUGUGGUU
3750 AGAGGAUGAU GCCAUGGAAC AGGGCGUAAG AGACUAUGUU GAACAACUAG
3800 GAAAUGCUUU CGGCUCAGGU UUCACCAAUC AAAUUUGUGA ACAGGUCAAC
3850 CUCCUCAAAG AGUCAUUGGU UGGACAGGAU UCUAUUCUGG AAAAAUCCCU
3900 UAAGGCUCUA GUUAAGAUUA UCUCAGCACU GGUCGUUGUA GUGAGAAAUC
3950 ACGAUGAUCU CAUAACGGUU ACCGCCACUC UAGCUUUAAU UGGUUGCACC
4000 UCUUCUCCGU GGCGGUGGCU CAAGCAGAAG GUGUCACAAU AUUAUGGAAU
4050 ACCCAGGGCC GAGCGACAAA ACAAUAGCUG GCUCAAGAAG UUUACUGAGA
4100 UGACCAACGC CUGCAAGGGC AUGGAGUGGA UAGCCAUAAA AAUUCAAAAG
4150 UUUAUUGAGU GGCUUAAAGU CAAGAUUCUG CCGGAAGUGA AGGAAAAACA
4200 CGAGUUCCUC AACAGGCUAA AGCAAUUACC ACUCCUAGAG AGCCAGAUUG
4250 CAACCAUAGA GCAGAGUGCA CCAUCGCAGA GUGAUCAAGA GCAACUCUUC
4300 UCCAACGUCC AGUACUUCGC CCAUUAUUGC AGAAAGUAUG CGCCAUUGUA
4350 CGCUGCCGAG GCGAAGAGAG UGUUCUCACU UGAGAAGAAA AUGAGCAACU
4400 ACAUACAGUU CAAGUCCAAA UGCCGUAUUG AGCCUGUAUG CUUACUCCUA
4450 CAUGGCAGCC CAGGGGCCGG AAAGUCCGUG GCCACCAACU UGAUUGGCAG
4500 AUCCCUCGCA GAAAAACUCA ACAGCUCUGU AUACUCCCUA CCACCAGACC
4550 CCGACCACUU UGACGGCUAC AAGCAGCAAG CGGUCGUGAU CAUGGAUGAC
4600 UUAUGCCAAA AUCCUGAUGG AAAAGAUGUC UCACUAUUUU GUCAGAUGGU
4650 UUCUAGCGUG GACUUUGUAC CACCGAUGGC UGCGCUAGAG GAAAAGGAA
4700 UCCUAUUUAC CUCCCCGUUC GUGUUGGCAU CAACCAACGC UGGGUCCAUC
4750 AAUGCACCCA CUGUGUCUGA CAGCAGAGCG CUCGCUAGGA GAUUCCACUU
4800 UGACAUGAAC AUUGAAGUCA UUUCUAUGUA CAGUCAAAAC GGCAAGAUCA
4850 ACAUGCCCAU GUCAGUUAAA ACAUGUGAUG AAGAGUGUUG UCCAGUUAAC
4900 UUCAAAAGGU GCUGCCCGUU GGUGUGUGGG AAGGCYAUGC AAUUCAUUGA
 
4950 UAGGAGAACU CAAGUUAGAU AUUCGCUGGA CAUGCUAGUU ACUGAAAUGU
5000 UUAGGGAGUA UAACCAUAGA CACAGUGUGG GAGCCACUCU UGAAGCUCUG
5050 UUCCAAGGGC CACCAGUCUA CAGAGAGAUC AAAAUCAGCG UCGCCCCAGA
5100 GACACCCCCA CCACCAGCUA UUGCUGAUUU ACUGAAAUCA GUGGACAGUG
5150 AAGCUGUGAG GGAAUACUGC AAGGAGAGAG GGUGGCUUGU GCCAGAGAUC
5200 AAUUCUACCC UACAAAUAGA GAAGCAUGUG AGUAGAGCAU UCAUAUGUUU
5250 ACAAGCCCUA ACCACGUUUG UUUCAGUUGC UGGUAUAAUA UACAUUAUUU
5300 ACAAAUUAUU UGCAGGUUUC CAAGGCGCCU ACACAGGGAU GCCCAACCAG
5350 AAACCUAAGG UGCCCACCCU GAGACAGGCC AAAGUACAGG GCCCAGCGUU
5400 UGAGUUCGCU GUGGCGAUGA UGAAAAGGAA CGCCAGUACA GUAAAAACCG
5450 AGUACGGUGA AUUCACCAUG CUUGGCAUUU ACGACAAGUG GGCGGUGUUA
5500 CCGCGCCACG CCAAGCCUGG CCCCACCAUC UUGAUGAAUG AUCAGGAAGU
5550 CGGCGUGUUG GAUGCCAAGG AACUAGUUGA UAAAGAUGGG ACAAAUCUAG
5600 AAUUGACUCU CCUGAAGCUC AACCGUAACG AAAAGUUCAG AGAUAUUAGG
5650 GGGUUUCUAG CAAGAGAAGA GGUUGAAGUG AAUGAAGCUG UCCUAGCAAU
5700 AAAUACAAGC AAAUUCCCUA ACAUGUACAU ACCAGUGGGC CAGGUGACUG
5750 ACUACGGGUU UCUGAACCUG GGAGGGACUC CCACGAAGAG AAUGCUCAUG
5800 UAUAACUUCC CAACUAGAGC AGGUCAGUGU GGAGGUGUCC UCAUGUCAAC
5850 AGGGAAAGUC CUGGGAAUAC AUGUAGGAGG GAAUGGACAU CAAGGGUUCU
5900 CAGCGGCACU CCUCAGGCAC UACUUCAACG AGGAGCAGGG UGAAAUAGAA
5950 UUCAUUGAGA GCUCAAAGGA CGCGGGAUUC CCUGUGAUCA ACACUCCCAG
6000 UAAGACAAAA UUGGAACCAA GUGUGUUUCA CCAGGUGUUC GAGGGCAACA
6050 AGGAACCAGC GGUCCUUAGA AAUGGGGACC CACGACUCAA AGCCAACUUC
6100 GAGGAAGCAA UCUUCUCCAA GUACAUUGGC AAUGUCAACA CGCAUGUAGA
6150 UGAGUACAUG UUGGAGGCUG UGGACCAUUA UGCAGGACAA CUAGCUACUC
6200 UGGACAUCAG UACGGAGCCC AUGAAGCUAG AGGACGCCGU GUAUGGUACA
6250 GAGGGGCUGG AAGCACUAGA CCUAACCACC AGUGCAGGCU ACCCUUACGU
6300 GGCCCUGGGC AUCAAGAAAA GAGAUAUUCU AUCUAAGAAG ACUAAAGACC
6350 UCACUAAGUU GAAGGAAUGC AUGGACAAAU AUGGCCUAAA UUUGCCAAUG
6400 GUAACCUACG UCAAAGAUGA GUUGAGAUCU GCUGAGAAGG UGGCCAAGGG
6450 AAAAUCCAGG CUUAUUGAGG CUUCUAGUCU CAAUGACUCA GUAGCAAUGA
6500 GGCAAACAUU UGGAAAUUUA UAUAAGACCU UUCACCUCAA CCCGGGCAUC
6550 GUUACGGGCA GUGCUGUUGG GUGUGAUCCA GAUGUGUUUU GGAGCAAGAU
6600 CCCUGUUAUG CUUGAUGGAC AUCUCAUAGC UUUUGACUAU UCAGGCUAUG
6650 ACGCUAGCCU CAGCCCAGUG UGGUUUGCAU GUUUGAAACU UCUCCUAGAG
6700 AAACUAGGGU AUACAAACAA GGAAACAAAC UACAUAGAUU ACCUCUGUAA
6750 UUCCCAUCAC CUGUAUAGAG ACAAGCACUA CUUUGUAAGA GGCGGUAUGC
6800 CAUCAGGGUG UUCAGGCACC AGCAUAUUUA AUUCCAUGAU UAACAACAUC
6850 AUAAUCAGGA CUCUCAUGCU GAAGGUUUAU AAAGGCAUUG AUUUGGACCA
6900 AUUCAGAAUG AUUGCCUAUG GGGAUGAUGU GAUUGCUUCC UAUCCGUGGC
6950 CUAUCGAUGC UUCGCUGUUA GCUGAAGCAG GAAAAGAUUA UGGUUUAAUC
7000 AUGACCCCAG CAGACAAAGG CGAGUGCUUC AACGAGGUAA CCUGGACGAA
7050 UGUGACCUUU CUGAAAAGGU ACUUUAGGGC AGAUGAGCAA UACCCAUUUC
7100 UGGUCCAUCC UGUUAUGCCA AUGAAGGACA UACAUGAGUC CAUUAGGUGG
7150 ACCAAAGAUC CCAAGAACAC ACAGGAUCAU GUGCGCUCGC UGUGCCUAUU
7200 GGCUUGGCAC AACGGGGAGC AAGAAUAUGA GGAGUUUAUU CGCAAGAUCA
7250 GAAGCGUGCC CGUUGGGCGC UGCUUGACCC UACCCGCUUU UUCAACACUG
7300 CGCAGGAAGU GGCUGGACUC CUUUUAAAAU UAGAGCAUAA UUAGUAAAUC
7350 AUAAUUGGCU UAACCCUACC GCAUGAACCG AACUUGAUAA AAGUGCGGUA
7400 GGGGUAAAUU CUCCGCAUUC GGUGCGG
Appendix 4: **Seq ID No 4**
   **Comparison of genomes of the unmodified (native) virus and modified virus**
   N: Unmodified (native) virus
   M: Modified virus 
1                                                    50
N  TTAAAACAGC CTGTGGGTTG TTCCCACCCA CAGGGCCCAC TGGGCGCTAG
M  .......... .......... .......... .......... ..........

   51                                                  100
N  CACACTGGTA TCACGGTACC TTTGTGCGCC TGTTTTATAT CCCCCTCCCC
M  .......... .......... C......... ........C. T.........

   101                                                 150
N  ACTGTAACTT AGAGAAATCA CATAAACGAT CAATAGAAGG CGCAGCACAC
M  .......... ...AG...G. ........G. ...C...TA. .T...T....

   151                                                 200
N  CAGCTGAGTC TTGACCAAGC ACTTCTGTTT CCCCGGACTG AGTATCAATA
M  ..A.....C. CC........ .........A ........C. ....A.....

   201                                                 250
N  GACTGCTCAC GCGGTTGAAG GAGAAAACGT TCGTTACCCG GCCAACTACT
M  .G......G. ....C..... .T........ .......... .....T....

   251                                                 300
N  TCGAGAAACC TAGTACCACC ATGAAAGTTG CGCAGTGTTT CGCTCAGCAC
M  .......... .......... .....G.... .....C.... .....C....

   301                                                 350
N  AACCCCAGTG TAGATCAGGT CGATGAGTCA CCGCATTCCC CACGGGCGAC
M  .......... .......... .......... .....C.... ..........

   351                                                 400
N  CGTGGCGGTG GCTGCGTTGG CGGCCTGCCT ATGGGGCAAC CCATGGGACG
M  .......... ......C... .......... .......... ..........

   401                                                 450
N  CTTCAATACT GACATGGTGC GAAGAGTCTA TTGAGCTAGT TGGTAGTCCT
M  .......... .......... .......... ........A. ..........

   451                                                 500
N  CCGGCCCCTG AATGCGGCTA ATCCTAACTG CGGAGCAAGT GCCCACAAAC
M  .......... .......... .......... ...G...... ..........

   501                                                 550
N  CAGTGGGTAG CTTGTCGTAA CGGGCAACTC TGCAGCGGAA CCGACTACTT
M  ........G. .......... ....T...C. .......... ..........

   551                                                 600
N  TGGGTGTCCG TGTTTCCTTT TATTCTTATT CTGGCTGCTT ATGGTGACAA
M  .......... .......... .......... .......... ..........

   601                                                 650
N  TTGAGAGATT GTTACCATAT AGCTATTGGA TTGGCCATCC GGTGACTAAC
M  .......... ...R...... .......... ......R..T .....G....
 
651 700
N AGAGCAATTA TATACCTCTT TGTTGGATTT ATACCACTTA ATTCCACTAA
M ........C. ...T...... .......... .........G .........G

701 750
N TTACAACACT CTGCTACACA TTATTTACTT AAAACCAAGA AGATGGGAGC
M .......... ........T. ......G... ...TA..... ..........

751 800
N ACAAGTATCA ACACAAAAAA CTGGTGCACA TGAGACCSGT TTGAGCGCTA
M .........G ........G. .......... C......... ..........

801 850
N ACGGAAGCTC CATCATTCAC TACACCAACA TCAATTACTA CAAAGATGCA
M .....CA... T......... ..T....... ....C..... ..........

851 900
N GCATCCAACT CAGCCAACAG GCAAGACTTC ACCCAAGATC CAGGCAAATT
M .......... .......... ...G..T... .....G.... ....T..G..

901 950
N CACCGAACCG GTCAAGGATA TCATGATCAA GTCAATGCCC GCCCTAAACT
M ...T...... .......... .......... A..G...... ..........

951 1000
N CACCGACCGT GGAGGAGTGT GGGTACAGTG ATAGGGTGAG ATCCATAACG
M .....T...C .........C ........C. .C........ ....C.....

1001 1050
N CTCGGCAACT CAACCATTAC CACTCAGGAG AGTGCAAATG TAGTTGTTGG
M .......... .......... ......A..A ........C. ..........

1051 1100
N CTATGGCGGG TGGCCAGAGT ACTTGAAAGA TGAAGAAGCT ACTGCGGAAG
M .......A.. .......... .......... .......... ..........

1101 1150
N ATCAACCAAC ACAACCCGAT GTAGCCACAT GCAGGTTTTA CACGCTGGAA
M ....G..... .......... ........R. .......C.. ....T.....

1151 1200
N TCCGTCCAGT GGGAGAAAAA TTCCGCTGGA TGGTGGTGGA AGTTCCCCGA
M .......... .......... .AG....... .......... ..........

1201 1250
N AGCACTTAAG GACATGGGCC TCTTTGGTCA AAACATGCAT TACCACTACC
M .......... .......... .......... G.......T. ........T.

1251 1300
N TCGGTAGAGC AGGCTACACT ATACACGTGC AGTGCAATGC ATCCAAATTC
M .......... .......... .....T.... .......C.. .........T

1301 1350
N CACCAAGGCT GTCTACTTGT TGTCTGTGTA CCTGAGGCTG AGATGGGGTG
M ..T..G.... .......... .......... .....A.... ..........

1351 1400
N TTCCAAAGTG GACGGTACTG TAAATGAGCA GGAATTGACG GAGGGTGAAA
M ....C.GAC. ...AAAGAG. .TGC..C.AT .A.CC.C... A.........

1401 1450
 
N CGGATATGAA GCTTGAACCC ACCAGAACCA CAGGCGTACG CCGAGTGCAA
M ...CGCAC.. .T.......A ....A..... ......GC.A .AC.......

1451 1500
N TCCGCAGTGT ACAACGCGGG TATGGGCGTC GGCGTGGGGA ACCTCACCAT
M ...AT..... G......... .......A.. .......... ..........

1501 1550
N CTTCCCTCAC CAGTGGATCA ACCTGCGCAC TAACAACTGT GCTACAATTG
M ..A....... .......... ..T....... ...T.....C ..........

1551 1600
N TGATGCCATA CATAAATAGT GTACCCATGG ATAACATGTT TAGGCACTAC
M .......G.. T......TCA .......... .......... ..........

1601 1650
N AACTTCACGC TAATGATGAT CCCATTTGCA CCCCTGGATT ACACCAACCA
M ..T....... .....G.... .......... .......... ...ATGC...

1651 1700
N AGCATCTACG TACGTACCTA TAACTGTCAC AATAGCACCA ATGTGTGCTG
M .......GA. .........G .......... ......C... ........A.

1701 1750
N AATACAATGG TTTGAGGCTC GTTACCTCGC AAGGGTTGCC AGTGATGAAC
M .......... ...A.....G .C.TA.CA.. .....C.... ....C.A..T

1751 1800
N ACACCGGGAA GCAATCAGTT CCTGACATCG GATGACTTTC AATCACCTTC
M .......... .......... TA........ .....T.... ....C.....

1801 1850
N GGCTATGCCA CAATTTGATG TGACTCCAGA CATGGACATC CCAGGTGAAG
M .......... .......... .......GC. .......... ..........

1851 1900
N TGAACAACCT CATGGAGATT GCAGAGGTTG ACTCGGTGGT ACCTGTTAAC
M ..C....... .......... .....A.... .T........ ..........

1901 1950
N AACAATGAGG CCAATCTGAA AAGCATGGAC GCATACCGCA TACCGGTGAA
M ....C..C.. ........C. .......... .....T.A.. ..GA......

1951 2000
N CRCAGGAAAT CAACAAGGTG AAAAGATATT TGGTTTCCAA ATACAACCCG
M .......... ..C....... .......... C.C......G ..........

2001 2050
N GGCTTGATTC AGTGTTTAAG AGAACACTGC TAGGTGAGAT GCTCAATTAT
M ....G..... .......... .......... .......AG. ..........

2051 2100
N TACACGCACT GGTCAGGGAG CATTAAGCTA ACATTTATGT TTTGTGGTTC
M ...G...... .......... .......... .....C.CA. ..........

2101 2150
N AGCAATGGCC ACGGGCAAAT TACTCTTAGC ATACTCACCA CCTGGCGCCG
M .......... ........GC .......... ......C... ..........

2151 2200
 
N ATGTACCGAC TAGCAGAAAG GAGGCAATGC TGGGAACCCA TGTCATCTGG
M ........G. .......... ....C....A .......... .A........

2201 2250
N GACTTTGGGC TGCAATCCAG TTGTGTTCTG TGTGTTCCAT GGATCAGCCA
M .....A.... .......... ...C.....A ...A...... .......T..

2251 2300
N GACACACTAC AGGTTGGTGC AGCAGGATGA GTACACCGGC GCCGGCTATA
M ......T..T C.CC.A.... .A........ .......A.. ......A..G

2301 2350
N TCACCTGCTG GTACCAAACA AGTATAGTGG TTCCACCCGG CACACCCAAA
M .......... ...T..G... G......... .......... .........C

2351 2400
N AAGTGTGTCA TCCTGTGCTT TGTGTCAGCG TGTAATGATT TCTCCGTGAG
M .........G .......... .......... ........C. ........C.

2401 2450
N CATGCTGAGT GACACACCAT TCATCGGCCA AACAGCACTG CTGCAGAGCC
M .......C.. .......... .......... ....A..... ..A..AG.TG

2451 2500
N CTGTGGAAGA AGCTGAAGAG AACGCAGTTG CACGTGTGGC TGACACAATT
M A.AC...C.T G..C.TCA.C ..T.....A. .CA.G..A.. ...T......

2501 2550
N GCCAGTGGGC CCAGCAACTC CGAGAGCGTT CCTGCACTAA CAGCAGTTGA
M .......... .......... .ACT...A.. .......... .C........

2551 2600
N GACTGGGCAC ACATCACAGG TAGTGCCTAG TGACACAATG CAAACAAGGC
M .......... .......... ...A...... ...T...... ......C...

2601 2650
N ATGTGAAGAA CTACCATTCG AGATCTGAGT CAACAATAGA GAACTTCCTT
M ....A..... .......... C.......A. .......... ..........

2651 2700
N AGCAGGTCCG CCTGTGTGTA TATTGAAGAG TACTATACCA ACACTGAAAC
M ...C....G. .......A.. .......... ....T..... .AGA.C..G.

2701 2750
N CAGACAAAAT TTATACATGT TGCCCACTAT AAATACTAGA TGGATGGTGC
M ...CGCC... AGG....... CATGG..... ....G..... A.........

2751 2800
N AATTGAGGAG AAAGTTTGAG ATGTTCACAT ACATGAGGTT TGACATGGAA
M ........C. .........A C......... .....C.... ...T.....G

2801 2850
N ATCACATTTG TTATCACTAG TAGACAACTG CATCGAACTA GCATGCCGCA
M .......... .......... .......... .C.G.G.... ....CG....

2851 2900
N GGACATGCCG GTACTGACAC ACCAAATCAT GTATGTACCA CCTGGTGGTC
M A......... CC........ .......... ....A....C ..........

2901 2950
N CAGTACCAAA CAGTGTGGAC GATTACGCAT GGCAAACTTC GACTAACCCA
 
M .......... ......AC. ....TT.... .......... ......T...

2951 3000
N AGTGTCTTTT GGACTGAGGG CAATGCCCCA CCGCGTATGT CCATACCATT
M ...A...... .......... .........C .......... ..........

3001 3050
N CATAAGCATA GGGAATGCAT ACAGCAACTT TTATGATGGG TCCTCGCACT
M T......... .......... .......... ......C..R .GG.......

3051 3100
N TCTTACAATA TGGGGTATAT GGCTACAACA CATTAAACAA CATGGGGAAA
M ...C....A. .........C ........TG .......... ......C...

3101 3150
N TTATACGTAC GCCATGTGAA CAACCACACA CCATACCAAA TGACCAGTAC
M .......C.. .......... ...AG..... ..G.....G. ..T.......

3151 3200
N GGTTAGTGTG TACTTTAAAC CCAAACATGT CAGAGCGTGG GTGCCGAGAC
M .A..C..... .......... ........A. .....T.... .....A....

3201 3250
N CACCACGTCT GTGCCCCTAC AAAAATGCAT GGAACGTTAA CTTTGAACCA
M ........T. .........T .TT..AT.TA .T........ ......C...

3251 3300
N ACAAACGTAA CTGATTCAAG ATCAAGTATC ACATATATTC CTGAGACGGT
M ..C...C... .......... .........A ......G.G. .A..C..TA.

3301 3350
N CAAACCAGAC CTATCAAAAG CTGGAGCTTT CGGCCACCAG TCCGGTGCTG
M .CGT..G..A G.CCGT.C.. .....AAA.. .......... ..........

3351 3400
N TTTATGTGGG TAACTACAGA GTGGTGAATA GGCACCTCGC CACGCACAAC
M ....C..... ...T...... A.A.....C. .......... ..........

3401 3450
N GACTGGCAAA ACTGTGTGTG GGAAGACTAC AACAGAGACC TCCTTGTGAG
M .......... .......... .......... .......... ..........

3451 3500
N CACCACCACA GCCCATGGGT GTGACACCAT AGCCAGATGC CAGTGCACAA
M ......T... .......... .......T.. .........T .........G

3501 3550
N CAGGCGTGTA CTTTTGTGCC TCAAGGAACA AACACTACCC AGTCACCTTT
M .......A.. T......... .......... ....T..... .........C

3551 3600
N GAGGGGCCAG GCCTGGTGGA AGTTCAGGAG AGTGAGTACT ACCCAAAAAG
M .......... ..T....... .......... ..C....... ..........

3601 3650
N ATACCAATCC CATGTGCTTC TAGCTGCAGG ATTTTCTGAA CCAGGCGATT
M .Y.T..G... ..C....... .......... .......... ..G.......

3651 3700
N GTGGTGGAAT CCTCAGGTGT GAACATGGTG TCATCGGTAT CGTCACCATG
M ....C..... ......A... C....C..C. .G........ ..........
 
3701                                              3750
N  GGTGGAGAGG GGGTCGTTGG GTTTGCCGAC GTCCGAGACC TACTGTGGTT
M  .......... .......... .......... ...A...... ..........

3751                                              3800
N  AGAGGATGAT GCCATGGAAC AGGGCGTAAG AGACTATGTT GAACAACTAG
M  .......... .......... .......... .......... ..........

3801                                              3850
N  GAAATGCTTT TGGCTCAGGT TTCACCAACC AAATTTGTGA ACAAGTCAAC
M  .......... C......... ........T. .......... ...G......

3851                                              3900
N  CTCCTCAAAG AGTCACTGGT TGGACAGGAC TCCATTCTGG AGAAATCCCT
M  .......... .....T.... .........T ..T....... .A........

3901                                              3950
N  TAAAGCCCTA GTTAAGATTA TCTCAGCACT GGTCATTGTA GTGAGAAATC
M  ...G..T... .......... .......... ....R..... ..........

3951                                              4000
N  ACGATGACCT CATCACAGTG ACTGCCACTC TAGCCCTCAT TGGTTGCACC
M  .......T.. ...A..G..T ..C....... ....TT.A.. ..........

4001                                              4050
N  TCTTCTCCAT GGCGGTGGCT CAAACAGAAA GTGTCACAAT ATTATGGAAT
M  ........G. .......... ...G.....G .......... ..........

4051                                              4100
N  ACCCATGGCT GAGCGACAAA ACAATGGCTG GCTCAAGAAG TTCACTGAGA
M  .....G...C .......... .....A.... .......... ..T.......

4101                                              4150
N  TGACCAATGC CTGCAAGGGC ATGGAGTGGA TAGCCATCAA AATTCAAAAA
M  .......C.. .......... .......... .......A.. .........G

4151                                              4200
N  TTTATTGAGT GGCTTAAAGT CAAGAT-CTA CCAGAAGTGT AGGAAAAACA
M  .......... .......... ......T..G ..G......A ..........

4201                                              4250
N  TGAGTTCCTC AACAGACTAT AACAACTACC ACTCTTGGAA GAGTCAGATT
M  C......... .....G...A .G...T.... ....C.A..- ...C......

4251                                              4300
N  GCCACCATAG AACAAAGTGC ACCATCGCAG AGTGACCAGG AGCAACTGTT
M  ..A....... .G..G..... .......... .....T..A. .......C..

4301                                              4350
N  TTCCAATGTC CAGTACTTCG CCCACTATTG CAGAAAGTAT GCGCCACTGT
M  C.....C... .......... ....T..... .......... ......T...

4351                                              4400
N  ATGCAGCTGA GGCAAAGAGA GTGTTCTCCC TTGAGAAGAA AATGAGCAAT
M  .C..T..C.. A..G...... ........A. .......... .........C

4401                                              4450
N  TACATACAGT TCAAGTCCAA ATGCCGTATT GAGCCTGTAT GTTTGCTCNT
M  .......... .......... .......... .......... .C..A...C.
 
4451                                              4500
N  ACATGGCAGC CCAGGGGCCG GAAAATCCGT GGCCACCAAC CTGATTGGCA
M  .......... .......... ....G..... .......... T.........

4501                                              4550
N  GATCACTCGC TGAAAAACTC AACAGCTCAG TGTACTCCCT ACCACCAGAC
M  ....C..... A......... ........T. .R........ ..........

4551                                              4600
N  CCAGATCACT TTGATGGCTA CAAACAGCAA GCGGTCGTGA TCATGGATGA
M  ..C..C.... ....C..... ...G...... .......... ..........

4601                                              4650
N  TCTATGCCAA AATCCTGATG GAAAAGATGT GTCATTGTTC TGTCAAATGG
M  CT........ .......... .......... C...C.A..T .....G....

4651                                              4700
N  TTTCCAGTGT GGACTTTGTA CCACCGATGG CTGCGCTAGA GGAGAAAGGC
M  ....T..C.. .......... .......... .......... ...A.....A

4701                                              4750
N  ATTCTGTTCA CCTCCCCGTT TGTCCTGGCA TCAACCAATG CTGGGTCCAT
M  ..C..A..T. .......... C..GT..... ........C. ..........

4751                                              4800
N  CAATGCACCA ACTGTGTCAG ACAGCAGAGC CCTCGCTAGG AGATTCCACT
M  .........C ........T. .......... G......... ..........

4801                                              4850
N  TTGACATGAA CATTGAAGTC ATTTCCATGT ACAGTCAAAA TGGCAAGATC
M  .......... .......... .....T.... .......... C.........

4851                                              4900
N  AACATGCCCA TGTCAGTTAA GACGTGTGAT GAAGAGTGTT GTCCAGTCAA
M  .......... .......... A..A...... .......... .......T..

4901                                              4950
N  CTTCAAGAGG TGCTGCCCGC TGGTGTGTGG AAAGGCCATG CAGTTCATTG
M  ......A... .........T .......... ......Y... ..A.......

4951                                              5000
N  ACAGAAGAAC TCAAGTTAGA TACTCGCTGG ACATGCTAGT TACTGAGATG
M  .T..G..... .......... ..T....... .......... ......A...

5001                                              5050
N  TTTAGGGAGT ACAACCACAG ACACAGTGTG GGAGCCACCC TTGAGGCTCT
M  .......... .T.....T.. .......... ........T. ....A.....

5051                                              5100
N  GTTCCAAGGG CCACCAGTCT ACAGAGAGAT CAAAATTAGT GTCGCACCAG
M  .......... .......... .......... ......C..C .....C....

5101                                              5150
N  AGACACCACC ACCACCAGCT ATTGCTGACT TACTGAAATC AGTGGACAGT
M  .......C.. .......... ........T. .......... ..........

5151                                              5200
N  GAAGCTGTGA GAGAGTACTG CAAAGAAAAG GGATGGCTTG TGCCAGAGAT
M  .......... .G..A..... ...G..G.GA ..G....... ..........

5201                                              5250
 
N CAACTCCACC CTACAAATTG AGAAGCATGT GAGCCGGGCA TTCATCTGTC
M ...T..T... ........A. .......... ...TA.A... .....A...T

5251 5300
N TGCAAGCACT AACCACGTTT GTTTCAGTTG CTGGAATAAT ATACATTATT
M .A.....C.. .......... .......... ....T..... ..........

5301 5350
N TACAAGCTAT TTGCAGGTTT CCAAGGCGCA TACACAGGGA TGCCCAACCA
M .....AT... .......... .........C .......... ..........

5351 5400
N GAAACCCAAG GTGCCCACCC TGAGACAAGC CAAAGTGCAA GGCCCAGCGT
M ......T... .......... .......G.. ......A..G ..........

5401 5450
N TTGAGTTTGC TGTGGCGATG ATGAAGAGGA ACTCCAGTAC AGTGAAAACC
M .......C.. .......... .....A.... ..G....... ...A......

5451 5500
N GAGTACGGTG AGTTCACCAT GCTTGGCATT TATGACAGGT GGGCGGTGTT
M .......... .A........ .......... ..C....A.. ..........

5501 5550
N ACCACGCCAC GCCAAACCTG GCCCAACCAT CTTGATGAAT GACCAGGAAG
M ...G...... .....G.... ....C..... .......... ..T.......

5551 5600
N TCGGCGTGTT GGATGCCAAG GAACTAGTGG ATAAGGATGG GACAAACCTA
M .......... .......... ........T. ....A..... ......T...

5601 5650
N GAACTGACAC TCCTGAAGCT CAACAGTAAT GAGAAGTTCA GAGACATCAG
M ...T....T. .......... ....C....C ..A....... ....T..T..

5651 5700
N AGGGTTCCTA GCCAAAGAAG AGGTTGAGGT GAATGAAGCT GTCCTAGCAA
M G.....T... ..A.G..... .......A.. .......... ..........

5701 5750
N TAAACACAAG CAAGTTCCCC AACATGTACA TACCAGTGGG CCAGGTGACT
M ....T..... ...A.....T .......... .......... ..........

5751 5800
N GACTACGGGT TCCTGAACCT GGGTGGGACG CCCACTAAGA GAATGCTCAT
M .......... .T........ ...A.....T .....G.... ..........

5801 5850
N GTACAACTTC CCCACTAGAG CAGGTCAGTG TGGTGGTGTC CTCATGTCCA
M ...T...... ..A....... .......... ...A...... ........A.

5851 5900
N CTGGGAAAGT CCTGGGGATA CATGTTGGTG GGAATGGTCA TCAAGGGTTC
M .A........ ......A... .....A..A. .......A.. ..........

5901 5950
N TCAGCAGCAC TCCTCAAGCA CTACTTCAAC GATGAACAAG GTGAAATAGA
M .....G.... ......G... .......... ..G..G..G. ..........

5951 6000
N GTTCATTGAG AGCTCAAAGG ACGCGGGGTT CCCTATCATC AACACACCCA
 
M A.......... .......... .......A.. ....G.G... .....T....

6001 6050
N GCAAGACCAA ACTGGAACCA AGTGTCTTCC ACCAG-TGTT TGAAGGCAAC
M .T.....A.. .T........ .....G..T. .....G.... C..G......

6051 6100
N AAAGAACCCA GCAGTCCTCA GAAATGGTGA TCCACGACTC AAAGCCAACT
M ..G.....-. ..G.....T. .......G.. C......... ..........

6101 6150
N TTGAGGAGGC CATCTTCTCC AAATACATTG GCAATGTCAA CACGCATGTG
M .C.....A.. A......... ..G....... .......... .........A

6151 6200
N GATGAGTACA TGTTGGAAGC TGTGGACCAT TATGCAGGAC AACTGGCTAC
M .......... .......G.. .......... .......... ....A.....

6201 6250
N TCTGGACATC AGCACGGAAC CAATGAAGCT GGAGGATGCC GTGTATGGTA
M .......... ..T.....G. .C........ A.....C... ..........

6251 6300
N CAGAGGGGCT GGAAGCACTA GACCTAACAA CCAGTGCAGG CTACCCTTAT
M .......... .......... ........C. .......... .........C

6301 6350
N GTTGCCCTGG GCATCAAGAA GAGAGACATC CTATCTAAGA AGACCAGGGA
M ..G....... .......... A.....T..T .......... ....T.AA..

6351 6400
N CCTCACTAAG TTGAAAGAAT GCATGGACAA GTATGGCCTA AACCTGCCAA
M .......... .....G.... .......... A......... ..TT......

6401 6450
N TGGTAACCTA TGTGAAAGAT GAGCTCAGAT CTGCAGAGAA GGTGGCCAAA
M .......... C..C...... ...T.G.... ....T..... .........G

6451 6500
N GGAAAATCCA GGCTTATTGA AGCTTCCAGT TTGAATGACT CAGTGGCAAT
M .......... .......... G.....T... C.C....... ....A.....

6501 6550
N GAGACAGACA TTTGGAAACC TGTACAAAAC CTTCCACCTC AACCCAGGCA
M ...G..A... ........TT .A..T..G.. ...T...... .....G....

6551 6600
N TTGTGACGGG CAGTGCAGTT GGGTGTGACC CAGATCTGTT TTGGAGCAAG
M .C..T..... ......T... ........T. .....G.... ..........

6601 6650
N ATACCAGTCA TGTTGGATGG ACATCTCATA GCTTTTGATT ACTCAGGCTA
M ..C..T..T. ..C.T..... .......... ........C. .T........

6651 6700
N TGATGCTAGC CTCAGCCCAG TGTGGTTTGC ATGTCTGAAA CTGCTCCTAG
M ...C...... .......... .......... ....T..... ..T.......

6701 6750
N AGAAGCTTGG GTACACACAC AAGGAAACAA ACTACATAGA TTACCTCTGC
M ....A..A.. ...T..A... .......... .......... .........T
 
6751                                              6800
N  AACTCCCACC ACCTGTACAG AGACAAACAC TACTTTGTGC GAGGTGGTAT
M  ..T.....T. .......T.. ......G... .........AA ....C.....

6801                                              6850
N  GCCATCAGGG TGTTCTGGCA CCAGCATCTT TAACTCAATG ATTAACAACA
M  .......... .....A.... .......A.. ...T..C... ..........

6851                                              6900
N  TCATAATCAG GACACTCATG CTGAAAGTGT ACAAGGGCAT TGACTTGGAC
M  .......... ...T...... .....G..T. .T..A..... ...T......

6901                                              6950
N  CAATTCAGGA TTATTGCCTA TGGTGATGAT GTGATTGCTT CCTACCCGTG
M  ........A. .G........ ...G...... .......... ....T.....

6951                                              7000
N  GCCCATTGAT GCTTCCCTGC TAGCTGAAGC AGGAAAAGAT TATGGTTTGA
M  ...T..C... .....G...T .......... .......... ........A.

7001                                              7050
N  TCATGACACC AGCAGATAAA GGAGAGTGCT TCAATGAAGT CAACTGGACG
M  .......C.. ......C... ..C....... ....C..G.. A.C.......

7051                                              7100
N  AATGTCACCT TCCTGAAAAG GTACTTTAGA GCAGATGAGC AATACCCATT
M  .....G.... .T........ .........G .......... ..........

7101                                              7150
N  CCTGGTCCAC CCTGTTATGC CCATGAAAGA CATCCATGAA TCTATTAGAT
M  T........T .......... .A.....G.. .........G ........G.

7151                                              7200
N  GGACCAAAGA TCCAAAGAAC ACCCAAGATC ATGTGCGCTC GCTGTGCCTA
M  .......... ...C...... ..A..G.... .......... ..........

7201                                              7250
N  TTGGCTTGGC ACAATGGGGA GCACGAATAT GAGGAGTTCA TTCGCAAAAT
M  .......... ....C..... ...A...... ........T. .......G..

7251                                              7300
N  CAGAAAGCGT GCCAGTTGGA CGCTGTTTGA CCCTACCTGC GTTTTCAACC
M  .....-.... ...C.....G .....C.... ........C.. T........A

7301                                              7350
N  CTGCGCAGGA AGTGGTTGGA CTCCTTTTAA AATAA-AGCA CAATTTAGTA
M  .......... .....C.... .......... ...T.G.... T....-....

7351                                              7400
N  AATTTGAATT GGCTTAACCC TACCGCACTA ACCGAACTAG ATAACGGTGC
M  ...CAT.... .......... ........TG. .........T. ....AA....

7401                                   7437
N  GGTAGGGGTA AATTCTCCGC ATTCGGTGCG GTCGAGG
M  .......... .......... .......... .------
 Sequence identity: 90,3%
Appendix 5: **Seq ID No 5**
   **Comparison of amino acid sequences of the unmodified (native) virus and modified virus**
   N: Unmodified (native) virus
   M: Modified virus 
1                                                  50
N  MGAQVSTQKT GAHETXLSAN GSSIIHYTNI NYYKDAASNS ANRQDFTQDP
M  .......... .......... .H........ .......... ..........

   51                                                 100
N  GKFTEPVKDI MIKSMPALNS PTVEECGYSD RVRSITLGNS TITTQESANV
M  .......... .......... .SA....... ....L..... ..........

   101                                                150
N  VVGYGGWPEY LKDEEATAED QPTQPDVATC RFYTLESVQW EKNSAGWWWK
M  .....R.... .......... .......... .......... ..........

   151                                                200
N  FPEALKDMGL FGQNMHYHYL GRAGYTIHVQ CNASKFHQGC LLVVCVPEAE
M  .......... .....L.... .......... .......... ..........

   201                                                250
N  MGCSKVDGTV NEQELTEGET DMKLEPTRTT GVRRVQSAVY NAGMGVGVGN
M  ....QT.KE. AAMN..K... AH.F...K.. .GHT...I.C .....I....

   251                                                300
N  LTIFPHQWIN LRTNNCATIV MPYINSVPMD NMFRHYNFTL MMIPFAPLDY
M  ...Y...... .......... .......... .......... .V........

   301                                                350
N  TNQASTYVPI TVTIAPMCAE YNGLRLVTSQ GLPVMNTPGS NQFLTSDDFQ
M  NA...E...V .......... ......AYQ. ....L..... ...M......

   351                                                400
N  SPSAMPQFDV TPDMDIPGEV NNLMEIAEVD SVVPVNNNEA NLKSMDAYRI
M  .......... ..H....... H......... .......TA. ..Q.....H.

   401                                                450
N  PVNXGNQQGE KIFGFQIQPG LDSVFKRTLL GEMLNYYTHW SGSIKLTFMF
M  E.....H... ...A...... .......... ..V....A.. ........T.

   451                                                500
N  CGSAMATGKL LLAYSPPGAD VPTSRKEAML GTHVIWDFGL QSSCVLCVPW
M  .......... .......... ..A...Q..M ...I...L.. .......I..

   501                                                550
N  ISQTHYRLVQ QDEYTGAGYI TCWYQTSIVV PPGTPKKCVI LCFVSACNDF
M  .......... .....S..NV .....G.... .....N...V ..........

   551                                                600
N  SVSMLSDTPF IGQTALLQSP VEEAEENAVA RVADTIASGP SNSESVPALT
M  ..R..R.... ....T...GD TDV.VN.... .......... ...T.I....

   601                                                650
 
N AVETGHTSQV VPSDTMQTRH VKNYHSRSES TIENFLSRSA CVYIEEYYTN
M .......... E......... .......... .......... .......FTK

651 700
N TETRQNLYML PTINTRWMVQ LRRKFEMFTY MRFDMEITFV ITSRQLHRTS
M DQDSA.R..S W...ARR... ......L... .......... ......PG..

701 750
N MPQDMPVLTH QIMYVPPGGP VPNSVDDYAW QTSTNPSVFW TEGNAPPRMS
M IA....P... ....I..... .....T.F.. .......I.. ..........

751 800
N IPFISIGNAY SNFYDGSSHF LQYGVYGYNT LNNMGKLYVR HVNNHTPYQM
M .......... ......W... S.N.....A ........A. ...KD.....

801 850
N TSTVSVYFKP KHVRAWVPRP PRLCPYKNAW NVNFEPTNVT DSRSSITYIP
M S..IR..... ..I.V..... ......IKSS ....D...L. ........V.

851 900
N ETVKPDLSKA GAFGHQSGAV YVGNYRVVNR HLATHNDWQN CVWEDYNRDL
M D.IR.EVRT. .K........ ......I... .......... .........

901 950
N LVSTTTAHGC DTIARCQCTT GVYFCASRNK HYPVTFEGPG LVEVQESEYY
M .......... .........A .......... .......... ..........

951 1000
N PKRYQSHVLL AAGFSEPGDC GGILRCEHGV IGIVTMGGEG VVGFADVRDL
M ...X...... .......... ......Q... .......... ..........

1001 1050
N LWLEDDAMEQ GVRDYVEQLG NAFGSGFTNQ ICEQVNLLKE SLVGQDSILE
M .......... .......... .......... .......... ..........

1051 1100
N KSLKALVKII SALVIVVRNH DDLITVTATL ALIGCTSSPW RWLKQKVSQY
M .......... ....X..... .......... .......... ..........

1101 1150
N YGIPMAERQN NGWLKKFTEM TNACKGMEWI AIKIQKFIEW LKVKIYQKCR
M ....R..... .S........ .......... .......... .....LPEVK

1151 1200
N KNMSSSTDYN NYHSWKSQIA TIEQSAPSQS DQEQLFSNVQ YFAHYCRKYA
M EKHEFLNRLK QLPLLE.... .......... .......... ..........

1201 1250
N PLYAAEAKRV FSLEKKMSNY IQFKSKCRIE PVCLLXHGSP GAGKSVATNL
M .......... .......... .......... .....L.... ..........

1251 1300
N IGRSLAEKLN SSVYSLPPDP DHFDGYKQQA VVIMDDLCQN PDGKDVSLFC
M .......... .......... .......... .......... ..........

1301 1350
N QMVSSVDFVP PMAALEEKGI LFTSPFVLAS TNAGSINAPT VSDSRALARR
M .......... .......... .......... .......... ..........

1351 1400
N FHFDMNIEVI SMYSQNGKIN MPMSVKTCDE ECCPVNFKRC CPLVCGKAMQ
 
M .......... .......... .......... .......... ..........

1401 1450
N FIDRRTQVRY SLDMLVTEMF REYNHRHSVG ATLEALFQGP PVYREIKISV
M .......... .......... .......... .......... ..........

1451 1500
N APETPPPPAI ADLLKSVDSE AVREYCKEKG WLVPEINSTL QIEKHVSRAF
M .......... .......... ........R. .......... ..........

1501 1550
N ICLQALTTFV SVAGIIYIIY KLFAGFQGAY TGMPNQKPKV PTLRQAKVQG
M .......... .......... .......... .......... ..........

1551 1600
N PAFEFAVAMM KRNSSTVKTE YGEFTMLGIY DRWAVLPRHA KPGPTILMND
M .......... ...A...... .......... .K........ ..........

1601 1650
N QEVGVLDAKE LVDKDGTNLE LTLLKLNSNE KFRDIRGFLA KEEVEVNEAV
M .......... .......... .......R.. .......... R.........

1651 1700
N LAINTSKFPN MYIPVGQVTD YGFLNLGGTP TKRMLMYNFP TRAGQCGGVL
M .......... .......... .......... .......... ..........

1701 1750
N MSTGKVLGIH VGGNGHQGFS AALLKHYFND EQGEIEFIES SKDAGFPIIN
M .......... .......... ....R....E .......... .......V..

1751 1800
N TPSKTKLEPS VFHQCLKATK NPAVLRNGDP RLKANFEEAI FSKYIGNVNT
M .......... ....VFEGN. E......... .......... ..........

1801 1850
N HVDEYMLEAV DHYAGQLATL DISTEPMKLE DAVYGTEGLE ALDLTTSAGY
M .......... .......... .......... .......... ..........

1851 1900
N PYVALGIKKR DILSKKTRDL TKLKECMDKY GLNLPMVTYV KDELRSAEKV
M .......... .......K.. .......... .......... ..........

1901 1950
N AKGKSRLIEA SSLNDSVAMR QTFGNLYKTF HLNPGIVTGS AVGCDPDLFW
M .......... .......... .......... .......... .......V..

1951 2000
N SKIPVMLDGH LIAFDYSGYD ASLSPVWFAC LKLLLEKLGY THKETNYIDY
M .......... .......... .......... .......... .N........

2001 2050
N LCNSHHLYRD KHYFVRGGMP SGCSGTSIFN SMINNIIIRT LMLKVYKGID
M .......... .......... .......... .......... ..........

2051 2100
N LDQFRIIAYG DDVIASYPWP IDASLLAEAG KDYGLIMTPA DKGECFNEVN
M .....M.... .......... .......... .......... .........T

2101 2150
N WTNVTFLKRY FRADEQYPFL VHPVMPMKDI HESIRWTKDP KNTQDHVRSL
M .......... .......... .......... .......... ..........
 
2151                                              2196
N  CLLAWHNGEH EYEEFIRKIR KRASWTLFDP TCVFNPAQEV VGLLLK
M  .........Q .......... SVPVGRCLTL PAFSTLRRKW LDSF--
 Sequence identity: 91%

## Claims

1. Modified enterovirus of ECHO 7 type **characterized by** a genome sequence that has at least 85%, preferably at least 95%, still more preferably at least 99% of sequence identical to Seq ID No 1 as set out on pages 18-20 of the description ; and
wherein changes in the sequence of the genome of the modified enterovirus are not larger than 0.7% after continuous propagation of the modified enterovirus in cell cultures for 12 months.

2. Modified enterovirus of ECHO 7 type, **characterized by** the genome sequence of Seq ID No 1 as set out on pages 18-20 of the description.

3. Method for manufacturing a modified enterovirus of ECHO 7 type by modification of native ECHO 7 virus, identified by genome sequence Seq ID No 2 as set out on pages 21-23 of the description, wherein the modification comprises conducting the virus adaptation in cancer cells, attenuated by an anti-cancer agent, subsequently passaging the modified virus in human embryonal fibroblast culture, propagating the modified virus in human melanoma cells, and subsequently passaging the modified virus in human embryonal fibroblast culture, optionally treated by ribavirin, and isolation and purification of the virus.

4. Method of claim 3, wherein the procedure of propagating the virus in cancer cells and subsequently passaging the modified virus in human embryonal fibroblast culture is repeated several times.

5. Method of claim 3 or 4, wherein the procedure of propagating the modified virus in human melanoma cells, and subsequently passaging the modified virus in human embryonal fibroblast culture is repeated several times.

6. Method of claim 3, 4 or 5, wherein the virus adaptation is conducted in cancer cells of at least two different cancers, such as human breast adenocarcinoma cells and gastric adenocarcinoma cells.

7. Method of any of claims 3-6, wherein the modification gives a modified enterovirus of ECHO 7 type, which is **characterized by** a genome sequence that has at least 85%, preferably at least 95%, still more preferably at least 99% of sequence identical to Seq ID No 1 as set out on pages 18-20 of the description ; and wherein in the modified enterovirus changes in the sequence of the genome are not larger than 0.7% after continuous propagation of the modified enterovirus in cell cultures for 12 months

8. Method of claim 7, wherein the modification gives a modified enterovirus of ECHO 7 type, which is **characterized by** a genome sequence of Seq ID No 1as set out on pages 18-20 of the description .

9. Method of claim 7 or 8, wherein the changes are one nucleotide replacements, which consist partly of mute mutations without change of corresponding amino acid.

10. The virus of claim 1 or 2 for use in treating oncological diseases.

11. The virus for use of claim 10, wherein the oncological disease is selected from the group consisting of: melanoma, gastric cancer, intestinal cancer, human breast cancer, prostate cancer, pancreatic cancer, lung cancer, kidney cancer, bladder cancer, lymphosarcoma, uterine cancer, angiosarcoma, rhabdomyosarcoma.

12. The virus for use of claim 10 wherein the oncological disease is melanoma.

## Patentansprüche

1. Modifiziertes Enterovirus vom Typ ECHO 7, **gekennzeichnet durch** eine Genomsequenz, die mindestens 85%, vorzugsweise mindestens 95%, noch mehr bevorzugt mindestens 99% sequenzidentisch mit Seq ID Nr. 1 ist wie auf Seiten 18-20 der Beschreibung dargelegt; und
worin Änderungen in der Genomsequenz des modifizierten Enterovirus nach kontinuierlicher Vermehrung des modifizierten Enterovirus in Zellkulturen über 12 Monate nicht größer als 0,7% sind.

2. Modifiziertes Enterovirus vom Typ ECHO 7, **gekennzeichnet durch** die Genomsequenz Seq ID Nr. 1 wie auf Seiten 18-20 der Beschreibung dargelegt.

3. Verfahren zur Herstellung eines modifizierten Enterovirus vom Typ ECHO 7 durch Modifikation eines nativen ECHO-7-Virus, identifiziert durch Genomsequenz Seq ID Nr. 2 wie auf Seiten 21-23 der Beschreibung dargelegt, worin die Modifikation die Durchführung der Virusadaption in durch ein Anti-Krebsmittel abgeschwächten Krebszellen umfasst, anschließendes Passagieren des modifizierten Virus in humaner embryonaler Fibroblastenkultur, Vermehren des modifizierten Virus in humanen Melanomzellen und anschließendes Passagieren des modifizierten Virus in humaner embryonaler Fibroblastenkultur, gegebenenfalls behandelt mit Ribavirin und Isolierung und Reinigung des Virus.

4. Verfahren nach Anspruch 3, worin die Maßnahme zur Vermehrung des Virus in Krebszellen und anschließendes Passagieren des modifizierten Virus in humaner embryonaler Fibroblastenkultur mehrfach wiederholt werden.

5. Verfahren nach Anspruch 3 oder 4, worin die Maßnahme zur Vermehrung des modifizierten Virus in humanen Melanomzellen und anschließendes Passagieren des modifizierten Virus in humaner embryonaler Fibroblastenkultur mehrfach wiederholt werden.

6. Verfahren nach Anspruch 3,4 oder 5, worin die Adaption des Virus in den Krebszellen von mindestens zwei verschiedenen Krebsarten durchgeführt wird, wie z.B. humanen Mamma-Adenokarzinom- und gastrischen Adenokarzinomzellen.

7. Verfahren nach einem der Ansprüche 3-6, worin die Modifikation ein modifiziertes Enterovirus vom Typ ECHO 7 ergibt, welches durch eine Genomsequenz gekennzeichnet ist, die mindestens 85%, vorzugsweise mindestens 95%, noch mehr bevorzugt mindestens 99% sequenzidentisch mit Seq ID Nr. 1 ist wie auf Seiten 18-20 der Beschreibung dargelegt; und worin Änderungen in der Genomsequenz des modifizierten Enterovirus nach kontinuierlicher Vermehrung des modifizierten Enterovirus in Zellkulturen über 12 Monate nicht größer als 0,7% sind.

8. Verfahren nach Anspruch 7, worin die Modifikation ein modifiziertes Enterovirus vom Typ ECHO 7 ergibt, welches durch die Genomsequenz Seq ID Nr. 1, wie auf Seiten 18-20 der Beschreibung dargelegt, gekennzeichnet ist.

9. Verfahren nach Anspruch 7 oder 8, worin die Änderungen Ein-Nukleotid-Austausche sind, die teilweise aus stummen Mutationen ohne Änderung der entsprechenden Aminosäure bestehen.

10. Das Virus nach Anspruch 1 oder 2 zur Verwendung in der Behandlung von onkologischen Erkrankungen.

11. Das Virus zur Verwendung nach Anspruch 10, worin die onkologische Erkrankung aus der Gruppe ausgewählt ist, bestehend aus: Melanom, Magenkrebs, Darmkrebs, humanem Brustkrebs, Prostatakrebs, Bauchspeicheldrüsenkrebs, Lungenkrebs, Nierenkrebs, Blasenkrebs, Lymphosarkom, Gebärmutterkrebs, Angiosarkom, Rhabdomyosarkom.

12. Das Virus zur Verwendung nach Anspruch 10, worin die onkologische Erkrankung ein Melanom ist.

## Revendications

1. Entérovirus modifié de type ECHO 7 **caractérisé par** une séquence du génome qui présente au moins 85 %, préférentiellement au moins 95 %, plus préférentiellement encore au moins 99 % de séquence identique à SEQ ID NO : 1 telle que présentée aux pages 18 à 20 de la description ; et
les changements dans la séquence du génome de l'entérovirus modifié n'étant pas de plus de 0,7 % après la propagation continue de l'entérovirus modifié dans des cultures cellulaires pendant 12 mois.

2. Entérovirus modifié de type ECHO 7, **caractérisé par** la séquence du génome de SEQ ID NO : 1 telle que présentée aux pages 18 à 20 de la description.

3. Procédé de fabrication d'un entérovirus modifié de type ECHO 7 par modification du virus ECHO 7 natif, identifié par la séquence du génome SEQ ID NO : 2 telle que présentée aux pages 21 à 23 de la description, la modification comprenant la conduction d'une adaptation du virus dans des cellules cancéreuses, atténuées par un agent anticancéreux, repiquage subséquent du virus modifié dans une culture de fibroblastes embryonnaires humains, propagation du virus modifié dans des cellules de mélanome humain, et repiquage subséquent du virus modifié dans une culture de fibroblastes embryonnaires humains, optionnellement traitée par de la ribavirine, et isolement et purification du virus.

4. Procédé selon la revendication 3, dans lequel la procédure de propagation du virus dans des cellules cancéreuses et de repiquage subséquent du virus modifié dans une culture de fibroblastes embryonnaires humains est répétée plusieurs fois.

5. Procédé selon la revendication 3 ou 4, dans lequel la procédure de propagation du virus modifié dans des cellules de mélanome humain, et de repiquage subséquent du virus modifié dans une culture de fibroblastes embryonnaires humains est répétée plusieurs fois.

6. Procédé selon la revendication 3, 4 ou 5, dans lequel l'adaptation du virus est conduite dans des cellules cancéreuses d'au moins deux cancers différents, telles que des cellules d'adénocarcinome du sein humain et des cellules d'adénocarcinome gastrique.

7. Procédé selon l'une quelconque des revendications 3 à 6, dans lequel la modification donne un entérovirus modifié de type ECHO 7, qui est **caractérisé par** une séquence du génome qui présente au moins 85 %, de préférence au moins 95 %, de façon encore davantage préférée au moins 99 % d'identité avec SEQ ID NO : 1 telle que présentée aux pages 18 à 20 de la description ; et dans lequel dans l'entérovirus modifié les changements dans la séquence du génome ne sont pas de plus de 0,7 % après la propagation en continu de l'entérovirus modifié dans des cultures cellulaires pendant 12 mois.

8. Procédé selon la revendication 7, dans lequel la modification donne un entérovirus modifié de type ECHO 7, qui est **caractérisé par** une séquence du génome de SEQ ID NO : 1 telle que présentée aux pages 18 à 20 de la description.

9. Procédé selon la revendication 7 ou 8, dans lequel les changements sont des remplacements d'un nucléotide, qui consistent partiellement en des mutations muettes sans changement de l'acide aminé correspondant.

10. Virus selon la revendication 1 ou 2 pour une utilisation dans le traitement de maladies oncologiques.

11. Virus pour une utilisation selon la revendication 10, la maladie oncologique étant choisie dans le groupe constitué de : mélanome, cancer gastrique, cancer intestinal, cancer du sein humain, cancer de la prostate, cancer pancréatique, cancer du poumon, cancer du rein, cancer de la vessie, lymphosarcome, cancer utérin, angiosarcome, rhabdomyosarcome.

12. Virus pour une utilisation selon la revendication 10, la maladie oncologique étant un mélanome.
